# EUROPEAN PATENT SPECIFICATION

(11) **EP 2 379 692 B1**
(45) Date of publication and mention of the grant of the patent: **05.12.2012**
(21) Application number: 09795686.6
(22) Date of filing: 22.12.2009
(51) Int. Cl.: C12M 1/00, C12M 3/00, F04B 43/00, F04B 43/073

(54) **BIOPHARMACEUTICAL PLANT IN A COLUMN**
BIOPHARMAZEUTISCHE ANLAGE IN EINER SÄULE
INSTALLATION DE PRODUITS BIOPHARMACEUTIQUES DANS UNE COLONNE

(30) Priority: 19.12.2008 DK 200801815; 29.10.2009 DK 200901165; 19.10.2009 DK 200901131; 21.10.2009 DK 200901142
(43) Date of publication of application: 26.10.2011
(73) Proprietor: Stobbe Tech A/s, 2840 Holte (DK)
(72) Inventor: STOBBE, Per, DK-2840 Holte (DK)
(74) Representative: ZBM Patents
(86) International application number: PCT/DK2009/000260
(87) International publication number: WO 2010/069320

(56) References cited:
- EP-A2- 0 155 237
- WO-A2-2009/069960
- US-A- 4 201 845
- US-A1- 2004 048 366
- US-A1- 2004 132 175
- US-B1- 6 844 187

## Description

### Technical Field

The present invention relates to a set of therapeutic material multifunctional processing highly flexible devices stacked in a column, hereby creating a process plant with extremely small foot-print and where preferably all processing devices are of single-use capabilities in order to obtain lowest possible costs. The present invention also relates to methods for continued operation of said bioreactor.

### Background of the Invention

Disposable bioreactors were considered a novelty, and perhaps even a passing trend, as recently as mid 90ties. As single-use bioreactors and filters have matured and begun to address most of these issues, their market acceptance has rapidly expanded. The development of disposable bioreactors and filters is, in many instances, driven by reduction in sterilisation and cleaning requirements, improved plant flexibility, reduced costs and faster time to market for the end product. Recently, all of these benefits have been well documented. The future hurdles to be overcome include the ability to add reliable, accurate, low cost sensors and pumping devices so that standards can be generated and the process repeatability readily documented.

A single-use stacked system provides a high degree of flexibility for biotech process design with a minimum of foot-print for reduced facility cost. It allows the practitioner to faster evaluate different configurations before deciding on the final design. Then, should the batch size be modified, or new requirements evolve, the system can be modified more easily to fit with new requirements. If also the single-use technology is available in all sizes like from small, clinical batches to full production scale further costs are reduced.

The main advantage of single-use are elimination of the preparation steps involved with stainless steel kit, such as cleaning, assembly, and disassembly, autoclaving and "clean-in-place" (CIP) or "sterilize-in-place" (SIP) operations, as well as associated labour, and validation costs and material costs.

So a Biopharmaceutical system, which is made up of different process step units such as for example a downstream pre-purification unit, an upstream media conditioning unit, and a pump unit kept together in a stack can be developed, build and implemented in weeks, rather than months.

Because of the reduced requirements for validation (including cleaning and sterilization) and the ease of manufacturing, process steps can be developed very quickly. Single-use technology will help speed up the process development and installation of manufacturing process line, which reduces the time to market the product produced in the biopharmaceutical system.

Preferably such a complete plant-in-a-stack should be suited for all Biopharmaceutical systems, which manufacture all types of drugs: monoclonal antibodies, vaccines, gene therapy, recombinant proteins, blood derivatives and small molecules, such as anticancer drugs. The present invention however is not limited to drug manufacturing. Also production of enzymes, vaccines, food ingredients or like is possible. Such technology is particularly well suited for CMOs (Contract Manufacturing Organizations), where a number of different products can be produced and the key issues are flexibility and avoiding cross-contamination.

Any size of bio factory involves typically the following process steps:
- Fermentation
- Product harvesting
- A first and second clarification, purification with membrane filters
- Sterile filtration with membrane filters
- polishing with membrane, resins chromatography
- storage

### Prior art

The Stax platform from the US based company Pall and the Zeta-Plus system from Cuno/3M offer both a holding system for bench-top as well as floor size for stacking of micro-filtration and depth filtration capsules.

There is no such thing available on the market or described in the patent literature, as disposable bioreactors or disposable fluid pumps, sensors, heating, conditioning, sparging devices, oxygenating devices integrated into capsules for stacking purposes into a bio factory.

Today's process steps are exclusively performed with individually and non-integrated bioreactors, filter, purification devices, pumps, valves, sensors, containers, etc.
- Reference is made to patent EP 317874A2 from Miles Inc in USA describing a method for continuous production and removal of a biological substance. Though depending on externally separation devices like outside the hollow fibre reactor and not in the column as the present invention explain.
- Reference is also made to US patent 6,544,424 by Refined Technologies, which describes a filtration system.

The documents, US 6 844 187 B1, US 2004/0048366 A1, EP 155 237 A2, US 2004/132175 A1 and US 4201845 A disclose bioreactor systems for cell culture with stacked modular parts.

### Description of the invention

The present invention solves the problem of how to improve the flexibility of a bioreactor by providing a number of capsules serving different purposes for the bio-reaction taken place in the bioreactor. The capsules are easily assembled in a stack. By selecting a particular combination of different capsules stacked in a predetermined sequence, a bioreactor providing the optimal solution for producing a particular chemical compound is easily developed.

The present invention also solves the problem of how to optimize the size of the bioreactor by providing a reactor with increased "compactness", which reduces the cost of manufacturing production facilities needed for industrial scale bio-fermentation.

The invention is a single-use Biopharmaceutical Plant or bio factory assembled from at least three individual capsules stacked into a column. The column operate on continues basis with in lifetime strictly opposite to the batch process. Such column comprises individual capsules which integrate individual devices such as;
1. Bioreactor capsules
2. Temperature conditioning capsules
3. pH, 02, and gas conditioning capsules
4. Down stream processing capsules, such as depth, surface, separation, ion exchange and purification devices
5. Pumping capsules
6. Sensors of re-usable and/or single-use capability

The above six groups each allow for many variations of performance and technical art though all being preferably of disposable character, which eliminates the costly CIP, SIP processes and the associated validation procedure. The stack of the present invention, which includes high cell density bioreactor capsules, further reduces the size requirements to the white room facilities. Operation mode based on the bio factory according to the present invention and its capsules integrated therein offers quite extraordinary possibilities, which so far are unknown in the industry.

The bioreactor according to the invention comprises

interconnected, stackable capsules, wherein one or more cell cultivation capsule(s) is separating two pumping capsules according to claim 1 capable of regulating the diversion of a feeding liquid to and from said cell cultivation capsule, said bioreactor optionally further comprising one or more pre-separation capsules, one or more sparging capsules, one or more diffusion capsules, one or more manifold capsules, wherein a pumping capsule is located at each end of the stack. The capsules present in between the two pumping capsules may be stacked individually in any order and any number, with the proviso that the top capsule and the bottom capsule in the column must be pumping capsules.

Another aspect of the present invention provides a bioreactor factory device comprising a production unit for cultivating biological cells and for producing one or more chemical compounds and optionally a down stream processing unit. The production unit comprises at least three capsules, which are interconnected and stacked. The three mandatory capsules of the production unit is a bioreactor capsule, and two pumping capsules designated first pumping capsule and second pumping capsule according to claim 1. The first pumping capsule is always positioned as the top capsule and the second pumping capsule is always positioned as the bottom capsule in the production unit. Consequently, a bioreactor capsule is always separating the first and the second pumping capsule. Furthermore, the first and the second pumping capsule are in operable and liquid contact with each other and are capable of regulating the diversion of a feeding liquid to and from the bioreactor capsule.

The number of bioreactor capsules present in the production unit may be any number, which is necessary in order to produce the amount of chemical compound during the period of time available for the manufacture. The number may lie in the range of 1 to 50 capsules such as for instance 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50.

In one embodiment the production unit of the bioreactor factory also comprises at least one conditioning capsule. This capsule may be located at any position between the first and the second pumping capsule, i.e. on any side of the bioreactor capsule(s) as well as in between bioreactor capsules in cases where more than one bioreactor capsule is present in the bioreactor unit.

In one embodiment at least one of the conditioning capsule(s) is a temperature regulating capsule. This temperature capsule may be capable of increasing the temperature of the liquid media flowing through the production unit and in other embodiments the capsule may be capable of decreasing the temperature of the liquid media flowing through the production unit. The number of temperature regulating capsules may take any number depending on the heating/cooling capacity of the capsule and dependent on the amount of heat to be generated or consumed due to the biochemical reaction taken place inside of the bioreactor unit. Typically the number of temperature regulating capsules lies in the range of 1 to 20, such as 1, 2, 3, 4, 5, 6, 7, 8, 9, 10, 11, 12, 13, 14, 15, 16, 17, 18, 19 or 20.

In one embodiment at least one of the conditioning capsule(s) is a diffusion capsule. This capsule takes the function of supplying oxygen to the media circulating through the bioreactor unit so that the oxygen consumed by the action of the microorganisms is continuously replaced by fresh oxygen. The number of diffusion capsules present in the bioreactor unit depends on the microorganisms in the bioreactor capsule and on the type of biochemical reaction occurring in the reactor. Typically the number of diffusion capsules lies in the range of 1 to 20, such as 1, 2, 3, 4, 5, 6, 7, 8, 9, 10, 11, 12, 13, 14, 15, 16, 17, 18, 19 or 20.

The conditioning capsule(s) may be a pH-regulating capsule. Such a capsule may be necessary in cases where acid or bases are produced during the biochemical reaction due to the action of microorganism, but also in cases where the microorganisms are very sensitive for any changes in the pH of the media, it may be necessary to monitor and regulate the pH continuously. Typically the number of pH-regulating capsules lies in the range of 1 to 20, such as 1, 2, 3, 4, 5, 6, 7, 8, 9, 10, 11, 12, 13, 14, 15, 16, 17, 18, 19 or 20.

The conditioning capsule(s) may be a combined diffusion and pH-regulating capsule, i.e. both supply of oxygen and regulation of pH takes place in the same capsule. This combination may be beneficial in cases where pH is regulated by supplying gases such as for example CO2 to the liquid media flowing through the reactor. The incorporation of two functions into one capsule also improves the compactness of the reactor. Typically the number of combined diffusion and pH-regulating capsules lies in the range of 1 to 20, such as 1, 2, 3, 4, 5, 6, 7, 8, 9, 10, 11, 12, 13, 14, 15, 16, 17, 18, 19 or 20.

In some cases it may be necessary or beneficial to supply fresh nutrient liquid to the production unit during operation. Consequently, the bioreactor may further comprise one or more capsules for providing fresh nutrient liquid to the production unit. It must be noted, however, the supply of fresh media may take place in any of the capsules, as long as the capsule is fitted with an inlet port, through which the fresh media can enter. Typically the number of capsules for providing fresh nutrient liquid lies in the range of 1 to 20, such as 1, 2, 3, 4, 5, 6, 7, 8, 9, 10, 11, 12, 13, 14, 15, 16, 17, 18, 19 or 20.

The bioreactor factory may further comprise one or more pumping capsule(s) located at any position between the first and the second pumping capsule. In cases where the total number of capsules in the production unit is significant, such as for example more than 5, it may be necessary to incorporate one or more pumping capsules in order to be able to pump the liquid through the production unit at essential same velocity at any place within the production unit. Typically the number of additional pumping capsules lies in the range of 1 to 20, such as 1, 2, 3, 4, 5, 6, 7, 8, 9, 10, 11, 12, 13, 14, 15, 16, 17, 18, 19 or 20.

In one embodiment the bioreactor factory device consists of at least one bioreactor capsule, at least two pumping capsules and at least one temperature regulating capsule.

In one embodiment the bioreactor factory device consists of at least one bioreactor capsule, at least two pumping capsules and at least one diffusion capsule.

The bioreactor factory device may consist of at least one bioreactor capsule, at least two pumping capsules and pH-regulating capsule.

The bioreactor factory device may consist of at least one bioreactor capsule, at least two pumping capsules and diffusion and pH-regulating capsule.

The bioreactor factory device may consist of at least one bioreactor capsule, at least two pumping capsules and at least one capsule for providing fresh media.

In one embodiment the bioreactor factory device consists of at least one bioreactor capsule, at least three pumping capsules and at least one down stream processing capsule.

The bioreactor factory device may consist of at least one bioreactor capsule, at least two pumping capsules, at least one temperature regulating capsule and at least one diffusion capsule.

The bioreactor factory device may consist of at least one bioreactor capsule, at least two pumping capsules, at least one temperature regulating capsule and at least one pH-regulating capsule.

The bioreactor factory device may consist of at least one bioreactor capsule, at least two pumping capsules, at least one temperature regulating capsule and at least one diffusion and pH-regulating capsule.

The bioreactor factory device may consist of at least one bioreactor capsule, at least two pumping capsules, at least one temperature regulating capsule and at least one diffusion and at least one capsule for providing fresh media.

In one embodiment the bioreactor factory device consists of at least one bioreactor capsule, at least three pumping capsules, at least one temperature regulating capsule and at least one down stream processing capsule.

The bioreactor factory device may consist of at least one bioreactor capsule, at least two pumping capsules, at least one diffusion capsule and at least one pH-regulating capsule.

The bioreactor factory device may consist of at least one bioreactor capsule, at least two pumping capsules, at least one diffusion capsule and at least one diffusion and pH-regulating capsule.

The bioreactor factory device may consist of at least one bioreactor capsule, at least two pumping capsules, at least one diffusion capsule and at least one capsule for providing fresh media.

In one embodiment the bioreactor factory device consists of at least one bioreactor capsule, at least three pumping capsules, at least one diffusion capsule and at least one down stream processing capsule.

The bioreactor factory device may consist of at least one bioreactor capsule, at least two pumping capsules, at least one pH-regulating capsule, and at least one diffusion and pH-regulating capsule.

The bioreactor factory device may consist of at least one bioreactor capsule, at least two pumping capsules, at least one pH-regulating capsule and at least one capsule for providing fresh media.

The bioreactor factory device may consist of at least one bioreactor capsule, at least three pumping capsules, at least one pH-regulating capsule and at least one down stream processing capsule.

The bioreactor factory device may consist of at least one bioreactor capsule, at least two pumping capsules, at least one diffusion and pH-regulating capsule and at least one capsule for providing fresh media.

The bioreactor factory device may consist of at least one bioreactor capsule, at least three pumping capsules, at least one diffusion and pH-regulating capsule and at least one down stream processing capsule.

The bioreactor factory evice may consist of at least one bioreactor capsule, at least two pumping capsules, at least one temperature regulating capsule, at least one diffusion, at least one pH-regulating capsule, and at least one capsule for providing fresh media.

The bioreactor factory device may consist of at least one bioreactor capsule, at least three pumping capsules, at least one temperature regulating capsule, at least one diffusion, at least one pH-regulating capsule, at least one capsule for providing fresh media, and at least one down stream processing capsule.

In order to be able to monitor parameters such as temperature, pH, oxygen concentration and the like, at least one of the capsules in the production unit is advantageously fitted with one or more sensors. Preferably such sensors are optical sensors.

In some embodiments the bioreactor factory device also comprises a down stream processing unit placed inside the column. The down stream processing unit comprises at least one down stream processing capsule and at least one pumping capsule. In other cases the bioreactor factory device the down stream processing unit may be placed outside the column. In yet other cases the bioreactor factory device do not comprise a down processing unit since such down stream processing is not necessary for the collection of the chemical compound of interest.

The production unit and the down stream processing unit may be placed in any order as compared to one another. Hence, in one embodiment the production unit is positioned on top of the down stream processing unit. In this case a pumping capsule must be positioned as the bottom capsule of the down stream processing unit. And in another embodiment of the present invention the down stream processing unit is positioned on top of the production unit, in which case a pumping capsule is positioned as the top capsule of the down stream processing unit. Hence, one pumping capsule is always positioned as the top capsule and another pumping capsule is always positioned as the bottom capsule in the bioreactor factory device.

The down stream processing capsule may perform any separation method which is known in the art. The choice of down stream processing process depends on the chemical compound of interest, which is to be isolated as well as the chemical and physical properties of the media from which the chemical compound is to be removed. In one embodiment, however, the down stream processing capsule is selected from the group consisting of a membrane filtration capsule, a separation capsule, a ion exchange capsule, a purification capsule, a chromatography capsule, an activated carbon capsule, and a packed bed capsule.

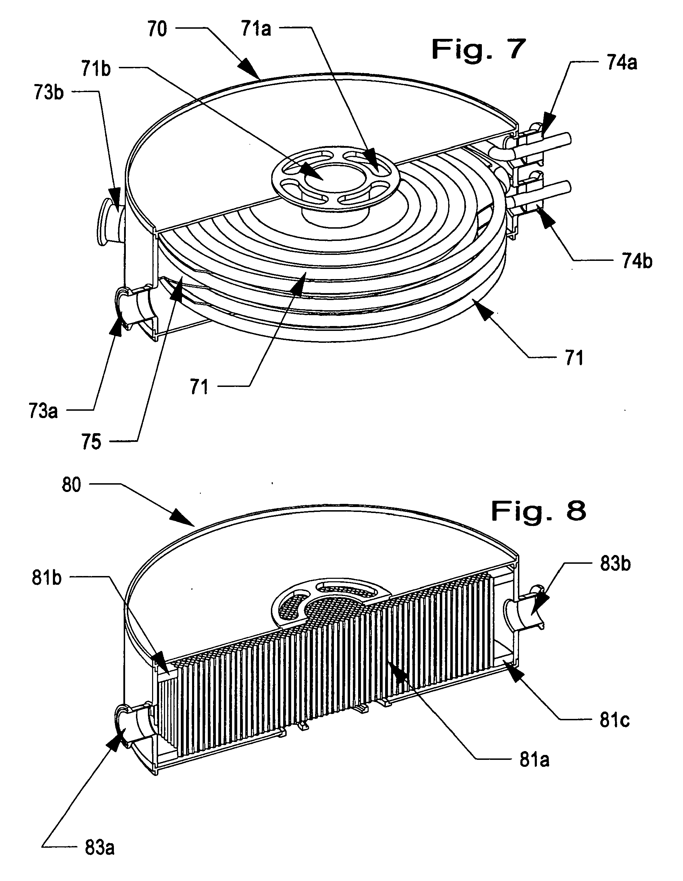

In a preferred embodiment, however, the down stream processing capsule comprises a flow-through matrix made up of a number of tubes through which the liquid to be processed is flowing. The compound of interest is separated from the liquid due to the action of the tubes, for example by being bound to the wall inside the tube by chemical or physical interaction. In a particular preferred embodiment the capsule is constructed similar to the capsule shown in figure 8.

Once the capsules are assembled into a stack the capsules may be supplied with means for exchanging liquids and information and the like. Hence, in one embodiment the capsules of the bioreactor is further provided with interlocking interface areas for exchange of fluid, electrical signals, electrical power, pneumatic signal exchange and/or vacuum signal exchange.

Once the capsules are assembled into a stack means for keeping the stack together during operation of the bioreactor factory would be advantageously. Consequently, in one embodiment the capsules of the bioreactor are further provided with interlocking means, such as latches, for keeping together the capsules in the stack.

It is well known that an important factor when performing microbiological reactions is that the bioreactor is sterile at the time of seeding the bioreactor. Hence in order to protect the capsules for being contaminated during assembling a sealing arrangement may be covering the capsules during storage and stacking the capsules and then removed after stacking but before a the capsules in the stack is forced together, preferably by use of the interlocking means.

The mode of operation is also flexible. Consequently, in one embodiment the capsules in the stack are placed in the horizontal position next to one another, and in another embodiment the stack of capsules are placed in the upright position vertical to one another.

Yet another aspect of the present invention provide a method for cultivating micro organisms and/or producing a chemical compound by use of a biochemical reaction taken place in the bioreactor as described above.

Definitions relevant for the present invention:
- Bioreactor or fermenter - a physical device, a container, a vessel which support biologically active environment and houses micro organism performing a process
- Bag - a flexible container made from clear plastic foil also used as bioreactor vessel
- Batch - a bioreactor to which no fresh medium is added and no cultured liquid removed
- Chemical compound -any compound, which can be formed in bioreactor, and includes, but are not limited to monoclonal antibodies, vaccines, gene therapy, proteins, recombinant proteins, blood derivatives and small molecules, such as anticancer drugs as well as enzymes, and food ingredients.
- Fed-batch - a bioreactor to which fresh medium is added and no cultured liquid removed until end of the process.
- Perfusion mode operation - operation principle for a Chemostat type of bioreactor, the media are continuously exchanged, fresh nutrients added and product is harvested throughout the culture period
- Cross flow mode operation - operation principle for a separation, filter unit
- Micro organism - microbes, cells such as; bacteria, fungi, algae, plankton, enzymes, animal, mammalian, tissue, yeast, plant, insect, protozoa, viruses, prokaryotic, eukaryotes, archaea, stem cells, micro animals, extremophiles.
- Anchorage dependent - cultivation of micro organism anchored onto a surface
- Suspension dependent - cultivation of micro organism, cell lines suspended in liquids
- Growth media, nutrient - fluid containing mostly water, carbon sources, various gases such as oxygen, vitamins, etc.
- Membrane - a layer of material, which serves as a selective barrier between two phases and remains impermeable to specific particles, molecules, or substances when exposed to the action of a driving force.
- Separation - dividing fluid borne particles of different size by membrane filtration into at least two separate fluid streams
- Filtration - mechanical method to separate solids from liquids or gases by passing the feed stream through a porous sheet such as a membrane, which retains the solids and allows the liquid to pass through.
- Purification - physical separation of a chemical substance of interest - typically performed downstream of the bioreactor in order to separate the product (like proteins) from waste (used and dead cells, damaged cells)
- Chromatography - Size Exclusion Chromatography - a device that holds chromatographic packing material, often the final step in a purification process. SEC works by trapping these smaller molecules in the pores of a particle. The larger molecules simply pass by the pores as they are too large to enter the pores.
- Disposable - products manufactured from organic materials preferably taken from the group consisting of thermo polymers, thermo setting polymers, and elastic polymers
- Porosity - is a measure of the void spaces in a material expressed in percent 0-100%
- Macro pore size - ranging 10-500 µm
- Meso pore size - ranging between 1-10 µm
- Micro pore size - ranging below 1 µm
- Non-woven - is a fabric-like flat porous sheets made from fibres, bonded together by chemical, mechanical, heat treatment, such as felt, which are neither woven nor knitted
- Honeycomb body - a one piece body with a multiple parallel small channel manufactured typically from ceramics
- Column - a series of capsules stacked on top of each other
- Capsules - a series of identical shaped containers of equal diameter, but with height according to the content, like one UH unit being 128 mm, two UH units being 256 mm
- Tri-clamp ports, flange - an industry method of assembling a hose to a container or pump, the Tri-clamp flanges are separated by a seal and kept together by the clamp with internal conical walls.

### Brief description of the drawings

Figure 1 - A column assembled from 3 bioreactor capsules and two pumping capsules, one pumping capsule at each end of the bio reactor capsule in opposite position facing the bioreactor.
Figure 2 - A pumping capsule with details of the diaphragm and two designs of valves
Figure 3 - A bioreactor capsule with ten fibrous matrix discs
Figure 4 - A down stream processing capsule with eighteen depth filter discs
Figure 5 - A manifold capsule with valves
Figure 6 - A conditioning capsule with heating element
Figure 7 - A conditioning capsule with cooling or sparging tubular element
Figure 8 - A diffusion capsule for various gases or filtration, separation purposes Figure 9 - An extended column comprising two bioreactor capsules, one down stream processing capsule, three pumping capsules, one manifold capsule, and two conditioning capsules
Figure 10 - The stack in principle, and principle fluid circuit diagram

### Detailed presentation of the invention

### Capsule physical design in general

Stacking may be performed by arranging the capsules next to each other in a pivoting holder system and the capsules in the stack may be interconnected by use of fluid exchange flange seals and kept together by use of clamps with such a force that the seals insure liquid tight performance. The numbers of capsules to be stacked are at least three and the stack could be as high as 2 meter.

The design of the preferred disposable capsules may be of tangential flow principle, radial flow principle, cross flow principle with symmetric or asymmetric porous bioreactor matrix design and either of round design, rectangular or combinations thereof.

The capsules may comprise channels for cooling or heating within the capsule structure, and the capsules may also comprise sanitary flange fittings such as tri-clamp or the common industrial 25 mm port for sensor, electrode connections.

The size of the capsules is not limited, but may be such as 12 or 16 inches or as the capsules shown in the figures, which are of 0442 mm in diameter. Sizes of the invented capsule designed column could match sizes in the industry like 12 or 16" being a diameter standard from 3Ms CUNO or Pall STAX product line. Capsules could even be of size like from 100 mm up to 1000 mm in diameter. For a bench-top column diameter of 150 mm and height of 400 mm results in a weight of less than 20 kilo. For a production column designed with 0442 mm diameter and 1500 mm height the total weight is less than 100 kilo and the bottom control capsule support the column to a wheel base, which also allows easy transportation within the production facility premises.

The capsule concept according to the present invention preferably combines communication along the column, such as wires, hoses, etc for transmission of signals for processing purposes or tasks for actuators to perform. A transmission line integrated in the capsules with plugs, and connectors, which integrate, when capsules are assembled.

Very compact and passive manifold capsules with no valves are commercially available with tri-clamp inlet and outlet flanges, which allow fluid connection to any of the capsules of the present invention.

### Assembling the capsules into the stack

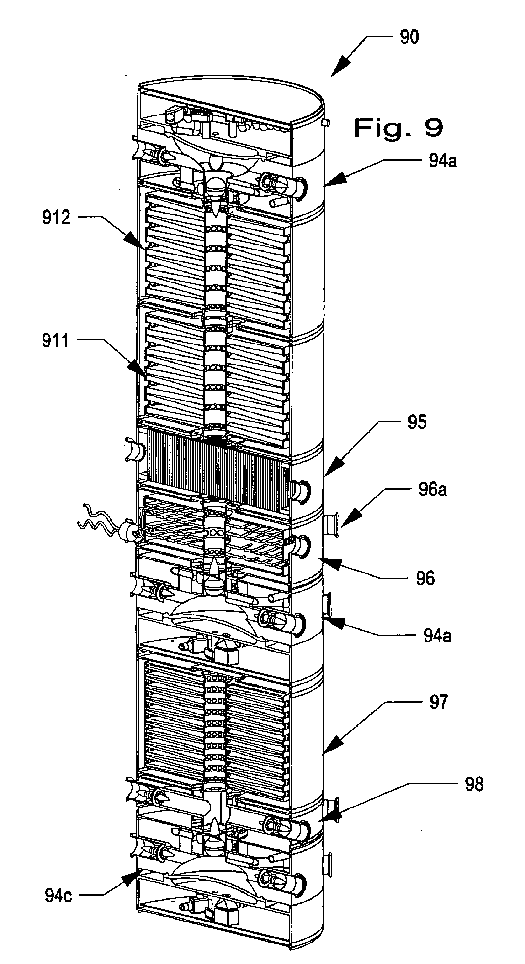

The bioreactor capsules, or cassettes, of the present invention may be combined in the same commercial filter pivoting holder system with similar sized filtration capsules, or cassettes for cell culture product clarification. The proposed set-up is a very compact solution and will enable the manufacturer to cut production cost and facility size further. Figure 9 illustrates a 1.7 meter high bio factory system with in total 9 capsules of which two are bioreactor capsules and one is a down stream processing capsule. The single-use, pre-sterilized capsule or cassette concept allows for fast exchange, development, variable capacity and small foot print.

Yet another variation of the present invention provides non round capsules or cassettes with at least one fluid inlet and at least one fluid outlet integrated within the capsules design. Such as the Plate & Frame design, an expression for old fashion assembling of a larger number of cassettes into a holder system with the typically square or rectangular plates, which typically operate in parallel. Also known as "filter presses" or "membrane plate filters" in the industry. A very common principle in the filtration industry and the design, column principle may be used for the bioreactor of the present invention.

The present invention also relates to the capsule design with interlocking mechanical clamp, means such as latches, which allow two or more individual capsules to be locked together fully independently of the known holding systems. The interlocking mechanical means is preferably based on two or more solid stainless steel rods, one rigid end plate and one movable top plate including a threaded arrangement to tighten the stacked capsules. The present invention eliminates the problems of contaminating the lab or production facility when being disassembled for disposal after use. Disassembling a stack of capsules filled with contaminant should in no way be exposed to the facilities, but rather be directly disposed of.

The single-use capsules assembled into larger set-up could be stacked within a horizontal pivoting holder system and the stack is then removed from this tool after assembly. In general the present invention avoids permanent use of stainless steel holding, pivoting systems.

Breakable seal in foil may cover the complete capsules end surface. Breakable seal in foil is covering each individual through going, fluid connector, manifold, flange for interconnections and protects the surface of the capsules from the surroundings. The capsules are stacked and the integrity seals are removed by pulling the seal in a radial direction exposing the two fluid connections to each other ready for interfacing. Use of seals on capsules is relevant on both bench-top systems and larger floor mounted systems. Bench-top systems could alternatively be delivered pre-assembled from the supplier.

Two capsules end face foil seals facing each other protect a sterilized and gas protected surface, internal devices and connections. The foil is double in a design where the outer foil is equipped with a handle and a 180 degree bend opposite of the handle. When the handle is pulled the sealing foil will slip the circumference attachment. When two capsule seals are removed simultaneously no contamination reaches the fluid connection and gas connection. Also a slight overpressure of protective gas in each capsule insures no contamination is sucked into the capsules.

A thin foil seal is integrated and glued on the circumference for protection purposes, and cover the entire end face and all the fluid flanges. Each capsule is to be connected without rotation to the next capsule controlled by a simple pair of male and female tracks located in a non-symmetrical mode on the capsule circumference outside the cover seal, which allow capsules of the column to be assembled without breaking the seals and prohibit incorrect orientation. The foil is to be broken when two capsules are correctly positioned towards each other exposing fluid connection, gas connection, electrical connectors rotational correct oriented towards the neighbouring capsule. The foils are removed simultaneously by dragging both foils of in a double mode configuration without exposing any end face to the local environment. After seal removal the two capsules need to be forced and pushed together for positive connection with latches.

For the ultimate very large bio factory system two or more different designed columns preferably work together and are connected internally with hoses via the tri-clamp port system. One column primarily assembled from bioreactor capsules, conditioning enclosed by at least one pumping capsule in each end. The sister column contains a number of desirable down stream processing capsules and two to three pumping capsules. The pump & valve control capsule allow via its communication bus to connect all data transfer with PLC or PC.

### 1. Bioreactor capsules

In principle the design and the mode of operation of the bioreactor capsule may take any form known within the art. Therefore, as used herein in one embodiment, the term "bioreactor" or "bioreactor capsule" also refers to a packed bed reactor chamber or a reactor which employs flow of biological cell culture and/or feeding liquid medium there through and which may be used for the growth of cell cultures or for packing with suitable support materials or matrices, such as, for example and without limitation, beads in the micrometer or millimetre scale (diameter), micro-spheres in general, micro-carriers, and the like, to which cells or cell colonies or cellular components can be attached or on which individual, isolated enzymes or enzyme systems can be immobilized for reaction with selected substrates.

In another embodiment the bioreactor of the present invention may also comprise a continuous, integrated membrane system. In the core of the bioreactor according to this embodiment of the present invention, the cells are confined between two membranes which form a cell chamber, at least one of which is preferably microporous and hydrophilic and permits free passage of nutrients present in a feeding liquid.

In one preferred embodiment the bioreactor capsules may comprise a plurality of permeable discs. The permeable disc of the present invention preferably does not contain or comprise a monolith, such as a ceramic thin wall, multi channel, and parallel channel monolith. Also, the permeable disc of the present invention preferably does not contain or comprise microspheres, such as e.g. essentially spherical beads.

The discs according to the present invention can be of any form and shape. In one embodiment the discs are circular with an optionally cut-out central portion to allow for the adaptation of a feeding tube or a collection reservoir in operable contact with the discs. The height (i.e. thickness) of the discs is preferably less than 500 millimeters, such as preferably less than 400 millimeters, for example preferably less than 350 millimeters, such as preferably less than 300 millimeters, for example preferably less than 250 millimeters, such as preferably less than 200 millimeters, for example preferably less than 150 millimeters, and preferably more than 1 millimeter, such as more than 2 millimeters, for example more than 5 millimeters, such as preferably more than 8 millimeters, for example preferably more than 10 millimeters, such as preferably more than 12 millimeters, for example preferably more than 14 millimeters, such as preferably more than 16 millimeters, for example preferably more than 18 millimeters, such as preferably more than 20 millimeters, for example preferably more than 22 millimeters, such as preferably more than 24 millimeters, for example preferably more than 26 millimeters, such as preferably more than 28 millimeters, for example preferably more than 30 millimeters, such as preferably more than 40 millimeters, for example preferably more than 50 millimeters.

The diameter of the discs (including the optionally cut-out, central portion) will depend on the reactor design, including the height of the discs, and the disc diameter is, in one embodiment, preferably less than 500 centimeters, such as less than 480 centimeters, for example less than 460 centimeters, such as less than 440 centimeters, for example less than 420 centimeters, such as less than 400 centimeters, for example less than 380 centimeters, such as less than 360 centimeters, for example less than 340 centimeters, such as less than 320 centimeters, for example less than 300 centimeters, such as less than 280 centimeters, for example less than 260 centimeters, such as less than 240 centimeters, for example less than 220 centimeters, such as less than 200 centimeters, for example less than 190 centimeters, such as less than 180 centimeters, for example less than 170 centimeters, such as less than 160 centimeters, for example less than 150 centimeters, such as less than 140 centimeters, for example less than 130 centimeters, such as less than 120 centimeters, for example less than 110 centimeters, such as less than 100 centimeters, for example less than 90 centimeters, such as less than 85 centimeters, for example less than 80 centimeters, such as less than 75 centimeters, for example less than 70 centimeters, such as less than 65 centimeters, for example less than 60 centimeters, such as less than 55 centimeters, for example less than 50 centimeters, such as less than 45 centimeters, for example less than 40 centimeters, such as less than 35 centimeters, for example less than 30 centimeters, such as less than 25 centimeters, for example less than 20 centimeters, and preferably more than 1 centimeter, such as more than 2 centimeters, for example more than 3 centimeters, such as more than 4 centimeters, for example more than 5 centimeters, such as more than 6 centimeters, for example more than 7 centimeters, such as more than 8 centimeters, for example more than 9 centimeters, such as more than 10 centimeters, for example more than 12 centimeters, such as more than 14 centimeters, for example more than 16 centimeters, such as more than 18 centimeters.

The permeable disc fibres preferably has, in one embodiment, an average diameter in the range of from 0.01 micrometer to preferably less than 100 micrometers, such as an average fibre diameter of from 0.05 to preferably less than 100 micrometers, for example an average fibre diameter of from 0.10 to preferably less than 100 micrometers, such as an average fibre diameter of from 0.15 to preferably less than 100 micrometers, for example an average fibre diameter of from 0.20 to preferably less than 100 micrometers, such as an average fibre diameter of from 0.25 to preferably less than 100 micrometers, for example an average fibre diameter of from 0.50 to preferably less than 100 micrometers, such as an average fibre diameter of from 1.0 to preferably less than 100 micrometers, for example an average fibre diameter of from 2.0 to preferably less than 100 micrometers, such as an average fibre diameter of from 4.0 to preferably less than 100 micrometers, for example an average fibre diameter of from 6.0 to preferably less than 100 micrometers, such as an average fibre diameter of from 8.0 to preferably less than 100 micrometers, for example an average fibre diameter of from 10 to preferably less than 100 micrometers, such as an average fibre diameter of from 12 to preferably less than 100 micrometers, for example an average fibre diameter of from 14 to preferably less than 100 micrometers, such as an average fibre diameter of from 20 to preferably less than 100 micrometers, for example an average fibre diameter of from 25 to preferably less than 100 micrometers.

The packing density of the permeable disc fibres is preferably, in one embodiment, in the range of from 100 to 5000 gram/meter2, such as from 100 to 200 gram/meter2, for example from 200 to 300 gram/meter2, such as from 300 to 400 gram/meter2, for example from 400 to 500 gram/meter2, such as from 500 to 600 gram/meter2, for example from 600 to 700 gram/meter2, such as from 700 to 800 gram/meter2, for example from 800 to 900 gram/meter2, such as from 900 to 1000 gram/meter2, for example from 1000 to 1100 gram/meter2, such as from 1100 to 1200 gram/meter2, for example from 1200 to 1300 gram/meter2, such as from 1400 to 1500 gram/meter2, for example from 1500 to 1600 gram/meter2, such as from 1700 to 1800 gram/meter2, for example from 1800 to 1900 gram/meter2, such as from 1900 to 2000 gram/meter2, for example from 2000 to 2100 gram/meter2, such as from 2100 to 2200 gram/meter2, for example from 2200 to 2300 gram/meter2, such as from 2300 to 2400 gram/meter2, for example from 2400 to 2500 gram/meter2, such as from 2500 to 2600 gram/meter2, for example from 2600 to 2700 gram/meter2, such as from 2700 to 2800 gram/meter2, for example from 2800 to 2900 gram/meter2, such as from 2900 to 3000 gram/meter2, for example from 3000 to 3100 gram/meter2, such as from 3100 to 3200 gram/meter2, for example from 3200 to 3300 gram/meter2, such as from 3300 to 3400 gram/meter2, for example from 3400 to 3500 gram/meter2, such as from3500 to 3600 gram/meter2, for example from 3600 to 3700 gram/meter2, such as from 3700 to 3800 gram/meter2, for example from 3800 to 3900 gram/meter2, such as from 3900 to 4000 gram/meter2, for example from 4000 to 4100 gram/meter2, such as from 4100 to 4200 gram/meter2, for example from 4200 to 4300 gram/meter2, such as from 4300 to 4400 gram/meter2, for example from 4400 to 4500 gram/meter2, such as from 4500 to 4600 gram/meter2, for example from 4600 to 4700 gram/meter2, such as from 4700 to 4800 gram/meter2, for example from 4800 to 4900 gram/meter2, such as from 4900 to 5000 gram/meter2, including any contiguous combination of the afore-mentioned sub-intervals.

Preferably, the volume of the open cells/pores of the matrix of the permeable discs/stacks is preferably less than 95% of the volume of the disc/stack itself, such as preferably less than 90%, for example less than 85%, such as less than 80%, for example less than 75%, such as less than 70%, for example less than 65%, such as less than 60%, for example less than 55%, such as less than 50%, for example less than 45%, such as less than 40%, for example less than 35%, such as less than 30%, for example less than 25%, such as less than 20%, and preferably more than 5%, for example more than 10%, such as more than 15% of the volume of the disc/stack itself.

The number of permeable discs/stacks present in the bio-reactor, or a capsule thereof, can be any number, preferably a number of from 2 to 100, such as 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100. The number of permeable discs/stacks is preferably an even number.

The bioreactor of the present invention may according to one embodiment comprise a porous matrix, one or more bio-reactor inlet port(s) and one or more bio-reactor outlet port(s),
wherein said matrix is suitable for anchoring and/or supporting the cultivation of living cells or micro organism in general,
wherein said matrix separates said one or more bio-reactor inlet port(s) from said one or more bio-reactor outlet port(s),
wherein said matrix comprises individual matrix sections each separated by a spacer section for diverting feeding liquid to or from said individual matrix sections.

Each of such "individual matrix section" can comprise a plurality, as for example at least 2, "individual matrix sub-sections", wherein each such sub-section is separated from another sub-section by a permeable layer comprising a plurality of openings exerting a biological cell retaining function, but not a feeding liquid retaining function, wherein the diameter of the openings of the permeable layer is smaller than the diameter of the biological cells to be cultivated, thereby creating a layer in between individual matrix sub-sections which is capable of diverting feeding liquid in a direction essentially perpendicular to the direction in which feeding spacers and drainage spacers are diverting feeding liquid to and from, respectively, the individual matrix sections.

The thickness of an individual "permeable layer", or the collective thickness of a plurality of "permeable layers" dividing an individual matrix section into a plurality of sub-sections, is preferably less than 10% of the thickness of the individual matrix section comprising said one or more permeable layer(s). The one or more permeable layer(s) preferably divide an individual matrix section into sub-sections having at least essentially similar thicknesses, i.e. no sub-section differs in thickness by more than 10% from any other individual matrix sub-section.

The permeable layer(s), when present, ensures that feeding liquid can flow from a feeding liquid spacer either through the individual matrix sections, or through the permeable layer, thereby further reducing and/or eliminating gradients of feeding liquid nutrients in the bio-reactor. Without being bound by theory, it is believed that the flow rate of feeding liquid along the permeable layer will be faster than the flow rate of feeding liquid through the individual matrix parts. However, it is also possible that under some practical conditions of bio-reactor operation, the flow along flow rate of feeding liquid along the permeable layer will be slower than the flow rate of feeding liquid through the individual matrix parts. Under such practical circumstances the feeding liquid being diverted along the permeable layer can be regarded as a supplemental source of feeding liquid useful for obtaining and sustaining optimal biological growth conditions.

In one aspect of the present invention there is provided a bio-reactor comprising a plurality of permeable discs comprising a matrix comprising a plurality of fibres defining a plurality of inter-connected, open cells or pores extending through at least part and preferably all of each disc,
wherein biological cells are capable of adhering to the fibres and capable of residing in at least some and preferably all of the inter-connected, open cells,
wherein the plurality of permeable discs are alternately separated by feeding spacer sections and drainage spacer sections, respectively, so that each feeding spacer section provides feeding liquid to each of a first set of two adjacently located, permeable discs and so that each drainage spacer section receives feeding fluid from each of a second set of two, adjacently located, permeable discs,
wherein each disc is contacted by one feeding spacer section and one drainage spacer section, respectively, on opposite sides of the disc,
wherein essentially all feeding liquid entering the disc from the side of the disc on which the feeding spacer section is located is diverted through the disc and exits the disc on the opposite side of the disc, where the drainage spacer section is located.

The first set of two adjacently located, permeable discs and the second set of two adjacently located, permeable discs have one disc in common when the feeding spacer section and the drainage spacer section are positioned next to each other in the bio-reactor, i.e. when they contact opposite surfaces of one and the same disc.

The overall flow of a feeding liquid through each disc is essentially in a direction which is perpendicular to the direction of the flow of feeding liquid along the feeding spacer section feeding said disc, i.e. the matrix material of the disc exerts a "cross-flow" effect on the feeding liquid. Likewise, once feeding liquid exits a disc it is diverted along the drainage spacer section in a direction which is also essentially perpendicular to the direction of flow of feeding liquid through the disc.

The open cells or pores of the matrix of the permeable discs are larger in diameter than the biological cells to be cultivated, thereby allowing said cells to reside in said open cells during all or part of the duration of the fermentation.

The bio-reactor can be fitted with a suitable pump in order to ensure the a desirable flow rate is obtained. The bio-reactor can also be fitted with sensors and a control unit for controlling the seeding of cells and the progression of the subsequent fermentation.

In another aspect of the present invention there is provided a bio-reactor comprising a plurality of permeable discs or stacks comprising a matrix comprising a plurality of fibres defining a plurality of inter-connected, open cells or pores extending through at least part and preferably all of each disc,
wherein biological cells are capable of adhering to the fibres and capable of residing in at least some and preferably all of the inter-connected, open cells,
wherein the plurality of permeable discs are alternately separated by feeding spacer sections and drainage spacer sections, respectively, so that each feeding spacer section provides feeding liquid to each of two adjacently located, permeable discs and so that each drainage spacer section receives feeding fluid from each of two, adjacently located, permeable discs,
i) a plurality of permeable discs comprising a matrix comprising a plurality of fibres defining a plurality of inter-connected, open cells or pores extending through at least part and preferably all of the disc,
   wherein biological cells are capable of adhering to the fibres and capable of residing in at least some of the inter-connected, open cells,
   wherein the plurality of permeable discs are alternately separated by feeding spacer sections and drainage spacer sections, respectively, so that each feeding spacer section provides feeding liquid to each of two adjacently located, permeable discs and so that each drainage spacer section receives feeding fluid from each of two, adjacently located, permeable discs,
ii) one or more inlet ports for diverting a feeding liquid to one or more feeding tubes of the reactor,
iii) one or more axially elongated feeding tubes comprising a plurality of openings through which feeding liquid can be diverted to each of the plurality of permeable discs,
iv) a plurality of elongated and optionally cross-linked feeding spacers in liquid connection at a proximal end thereof with the one or more feeding tubes and extending in an essentially radial direction therefrom,
   wherein feeding liquid can be diverted from said one or more feeding tubes to said plurality of feeding spacers through said holes of said one or more feeding tubes,
   wherein the distal end of each feeding spacer is not connected to an outlet port for diverting feeding liquid to a collection reservoir,
   wherein each feeding spacer separates two adjacently positioned permeable discs,
v) a plurality of elongated and optionally cross-linked drainage spacers in liquid connection at a distal end thereof with one or more outlet ports for diverting feeding liquid to a collection reservoir,
   wherein the plurality of drainage spacers are located essentially in parallel to the plurality of feeding spacers,
   wherein feeding liquid can be diverted from each of said plurality of feeding spacers and through a pair of neighbouring, permeable discs separated by a feeding spacer to one or more drainage spacers,
   wherein the distal end of each drainage spacer is not connected to a feeding tube hole,
   wherein each but end-positioned drainage spacer(s) separates two adjacently positioned permeable discs,
vi) a wall section comprising a plurality of collection reservoir outlet ports,
   wherein each collection reservoir outlet port is in liquid contact with a collection reservoir, and
vii) a collection reservoir for collecting feeding liquid diverted to said collection reservoir from the permeable discs via drainage spacers and through said collection reservoir outlet ports.

### 2. Temperature conditioning capsules

A number of different designs of heat exchangers for cooling or heating, which are commercial available for temperature regulations, may be used and incorporated into the bioreactor stack of the present invention in order to control the temperature of the liquid flowing through the bioreactor.

The fermentation or micro organism activity is typically an exothermic process generating heat. The volume/surface ratio of the reactor is decisive for weather heat must be applied or removed. For insect cells the required process temperature lies in the range of 27-28 degree Celsius and for mammalian cells the needed temperature is around 37 degree Celsius. Media contact either by flat surface or tube contact in a capsule with external connections for internal sterility and less demand to external conditions. The fluid from an external temperature conditioning devise is constantly circulated in the fluid-conditioning capsule controlling the stack temperature. More than one temperature conditioning capsule may be suited for a high stack.

Temperature regulation could also be done with integrated Peltier elements, which are able to either cool or heat depending on how the DC poles are connected to the two element connections.

Heating is performed preferably by the heating element cast located in suitable plastics. Flat cupper bands located between two connection points and oriented in a spiral forms the resistant heating element. Alternatively, the product Bekinox, which is available from the Belgium company Bekaert, could also be incorporated in the conditioning capsule.

### 3. Gas conditioning capsules

Unlike bacterial cultures, which are aerated with air, cell cultures are aerated with multiple gases such as oxygen, nitrogen, CO₂, and air in a mixture, which is specific for each type of micro organism and the specific fermentation process. Gas composition is regulated by sensitive control systems, and an optimum gas atmosphere should be formed and maintained for the fermentation and manufacture of the product. During cell proliferation the pH of the medium changes and keeping pH constant is vital for many production processes and therefore it must be controlled. pH control could be done by adding acids and bases as it is done in bacterial fermentations but preferably addition of CO₂ is controlling the pH.

The supply gases are usually accomplished from an external mounted gas-mixing station. In the invented device the dosage of each gas is controlled by the comprehensive control capacity included in the pump & valve controller. As the gas mixing takes place within the diffusion capsule, the delay time from long hoses from an external mounted gas mixing station is eliminated. The pump & valve controller regulates pH by adding dosages of CO₂ and the O₂ control regulates the aeration with air or Oxygen and Nitrogen. Thus the optimum gas supply for the micro organisms is facilitated for every phase of the fermentation.

Two principles are well known for adjustment of the oxygen concentration in the circulated media:
- Diffusion capsule - capable of exchanging O₂ and CO₂ through a membrane which is permeable to gas and impermeable to liquids. Such membranes have fluid on one side of the membrane and pressurized sterilized gas on the other side. Gas transfer occurs by diffusion in either direction. Figure 8 illustrate such invented capsule.
- Sparging air capsule - with sterilized air or O₂ by pressure forced through a micro porous diffuser membrane by applying a pressure and thereby creating micro bubbles ranging in size from nano meters to 2,000 µm in size. Separate pressure sensors connected to a gas chamber insure positive overpressure, which avoid fluids passing from pumping chamber into the gas chamber through the porous wall. In one aspect the sparging of gas into a fluid is supported by ultra sound, vibration devices helping break up bubbles. Both Figure 7 and 8 illustrates such invented capsule.

The amount of dissolved Oxygen (dO) is limited by the low solubility of oxygen in aquatic buffers and media. In general cells demand 10-14 to 10-13 mole oxygen per cell per hour and therefore efficient oxygen transfer for high cell density fermentations is critical. Although sparging with air/oxygen bubbles can efficiently transfer oxygen to the medium, such bubbles can cause severe damage to the cells. Cultivated mammalian cells are damaged by air-liquid interfaces due to the interaction with gas bubbles. Therefore a bubble free diffusion capsule with gas transfer by diffusion is most efficient. Other micro organisms are much more tolerant to bubbles. The diffusion materials are typically:
- Hollow fibre membranes, which are well recognised for the filtration performance. Hollow fibre membranes are typically of hydrophobic character with membrane pores ranging from 1-500 nm in diameter, typically 10-100 nm. The fibres may have outside diameter ranging from 1-10 mm. The membranes are bundled, stacked between two epoxy end covers typically cast further into a rigid plastic tube integrating the porous device.
- Gas-permeable hoses/tube/fibre capsule such as hollow micro pore, porous tubes like manufactured from silicone rubber, PFTE.

Examples of sparging materials are:
- Porous products, tubes made from glass and plastics often based on particles linked, fused together into the porous structure often have pore size ranging from 10 µm and up.
- Porous products, tubes made from ceramics offer a wide variety of pore sizes ranging from nano meters and up.
- Porous plastic membranes range from nano size pores and up.

The media supply capsule receives new nutrient, and optionally various gases preferably in concentrated form and is supplied dilute into the re-circulating fluid according to the information send from sensors on a real-time basis and prediction all controlled by the PLC within the pump & valve controller.

The pH and O₂ adjustment may take place in separate individual capsules or both adjustments may take place in the same capsule. Supply of fresh nutrient liquid may take place in any of the capsules in the biofactory or the supply may occur in a separate capsule.

### 4. Down-stream processing capsules, such as depth separation, surface separation, ion exchange or other purification devices

Any down stream processing method and design known in the art may be incorporated into the bioreactor of the present invention. In this step the product of interest is isolated from the liquid leaving the production unit.

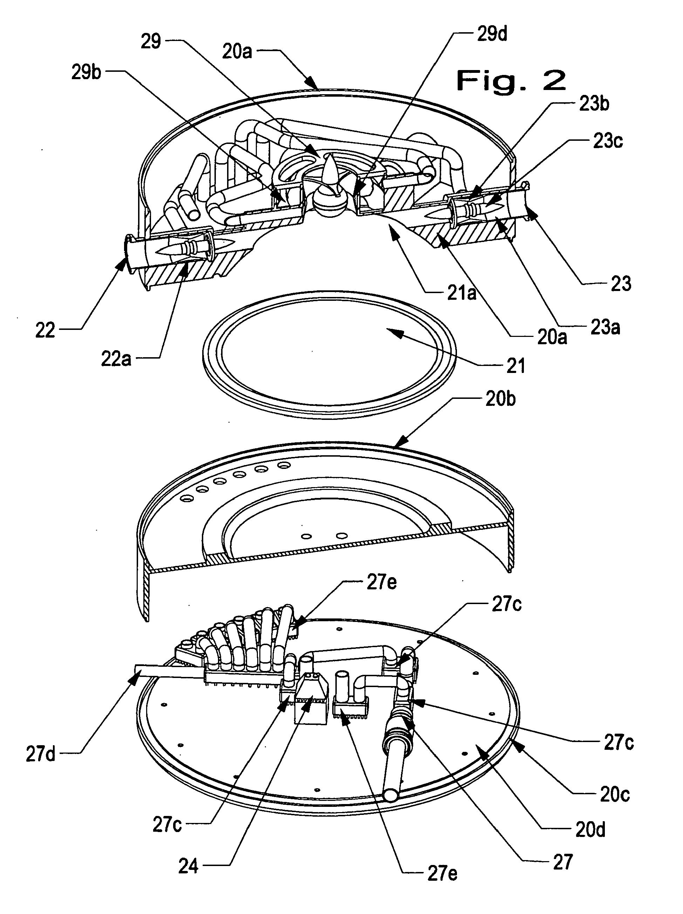

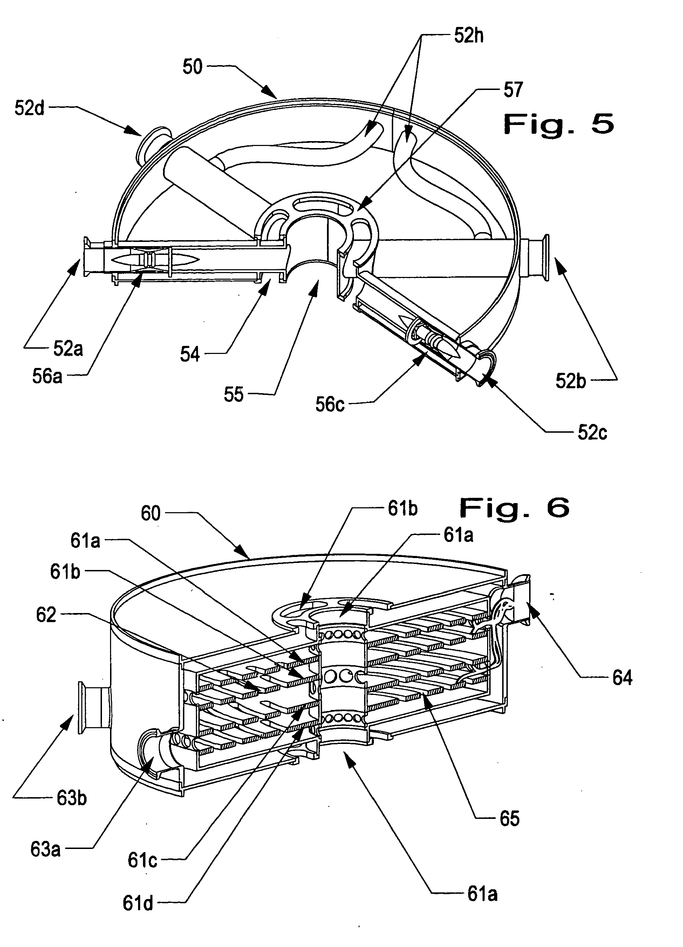

Hence, the first step after the bioreactor (production unit) of the retentate flow is the down stream processing or pre-clarification or product clarification. Many desirable principles are well-known without being necessary practical to utilize. Figure 8 illustrate such a situation as the invented multi-channel containing capsule combined with pumping capsule (Figure 2) and manifold capsule (Figure 5) facilitates operation after the Tangential Flow Filtration (TFF), also known as Cross Flow Filtration (CFF) principles. Which is a rapid and efficient method for filtration and separation of solutions containing bio molecules, or particles such as viruses, bacteria or cellular material.

In a first process the product flow (such as feed after bioreactor) is directed tangentially along the surface of a hollow fibre membrane with most of the solution circulated back to the feed storage as retentate. The high velocity cross flow creates a pressure drop, which forces some of the feed solution and dissolved molecules that are smaller than the pores in the membrane, through the membrane filter. The solution that passes through the membrane is referred to as filtrate or permeate. Molecules or particles larger than the membrane pores are retained in the feed solution and effectively concentrated. The fluid dynamics of cross flow filtration reduces membrane fouling and maintains filtration rates (flux) for a longer period of use, thus increasing membrane throughput (greater capacity) compared to traditional cartridge (direct flow) filtration.

In a second cleaning process step the high velocity feed solution along the membrane surface acts to 'sweep' the surface, reducing concentration polarization (product concentration at the membrane surface). It also prevents build-up of foulants that can plug the pores at the membrane surface.

In an additional third cleaning process the membrane module is exposed to a fluid pulse in the opposite direction, a back-flushing pulse, which helps remove potential build up on the feed flow side of the membrane. All such methods are well known within the filtration industry.

Also the option of sterilization with 200 nm filters or better are available on the marked from many vendors, such as the Fluorodyne® EX Filter Series from US company Pall. The new products from CUNO, the Zeta Plus™ EXT line of depth filters are supplied with two types of media that perform complementary purifications during post-cell culture harvest. Composed of cellulose and a positively charged resin, EXT SP is an open-grade medium that serves as an upstream or pre-filter that can replace centrifugation for small (<200 L) batches. EXT ZA media are of tighter grade and hold a strongly positive resin for removal of host cell proteins and DNA. Down stream processing capsules may easily be incorporated into the invented column present in the bioreactor and single-use pumps may also be present.

Centrifuging is less of a required process as the producing micro organism is kept stationary, contained in bioreactor capsule. Down stream the bioreactor a depth filter may efficiently trap, capture the low levels of cell debris, impurities in the harvest stream. Modern depth filters can be effectively employed in an adsorptive capacity for the capture of soluble impurities in addition to their conventional use in clarifying particle moderate fluid streams. The present invention is designed to replace energy-consuming centrifuges in a biotech process. Centrifuges are after all not single use, difficult to scale up and require a high effort for cleaning, cleaning validation and maintenance.

One of several final steps in down stream processing is the popular chromatography method for product contaminant removal and capture of large target molecules.

At least three methods are known:
1. Liquid chromatography ion-exchange to separate proteins take advantage of the fact that the molecules is suspended in a solution (mobile phase) and will interact with the chromatography media (stationary phase).
2. Packed bed ion-exchange chromatography with capsules for stacking options are well-known and are typically based on a solid absorbent resin (functionalised porous beads or gel polymer) such as the hydrophilic dextran, but zeolites, montmorillonite and diatomaceous earth have also been used. According to the difference of the interaction forces between the protein and adsorbent, different protein is bound differently by the adsorbent. When other buffers are used to replace the protein, the proteins will be eluted at different velocity: the less the interaction between the adsorbent and the proteins, the sooner they will be eluted. Then, proteins can be separated according to the sequence of their elution. Ion exchangers are either cation exchangers that exchange positively charged ions (cations) or anion exchangers that exchange negatively charged ions (anions). There are also amphoteric exchangers that are able to exchange both cations and anions simultaneously. However, the simultaneous exchange of cations and anions can be more efficiently performed in mixed beds that contain a mixture of anion and cation exchange resins, or passing the treated solution through several different ion exchange materials.
3. Membrane chromatography ion-exchange is well within the reach of becoming disposable and hereby possible to introduce into the invented column. In membrane chromatography processes, the transport of solutes to their binding sites take place predominantly by convection and the pore diffusion is very small as compared to the packed bed column, and thereby the mass transfer resistance is tremendously reduced. The binding capacity of membrane adsorbents for large size protein is much higher than that of traditional beads in packed beds. Membrane chromatography has higher capture efficiency and higher productivity than column chromatography and shows the most promising industrial applications for the recovery, isolation and purification of proteins and enzymes.

Several companies offer perfusion chromatography media to be included in capsules of and integrated in the present invention for the small foot-print, which is one of the key elements of the invention. Some chromatography media is even suitable to apply on micro porous membranes as coatings combining several features into one device.

Chromatography separation, which relies on membrane separation, depends partly on pressure drop, as to the extremely small pore size, somewhat higher, ranging pressures of 100-300 kPa. This technique and method is well within the reach of being of disposable character and hereby possible to introduce into the invented column.

Another final purification step of increased interest is separation by suspension crystallization based on freezing with refrigerants equipment temperature capacity ranging to minus 60 degree Celsius. The crystalliser converts the feed into a slurry of pure product crystals and the residual mother liquor. Typically the cooling takes place in one column being a part of a loop where the crystal growth and separation takes place in a separate column before traditional mechanical device compresses the slurry to remove the mother liquor and form a packed crystal bed. This bed consists of the pure product crystals surrounded by some residual mother liquor. The equipment for this process is large, depends on materials, which eliminate that the technology becomes easily of disposable character. Companies like GEA, Sulzer and other offer such equipment.

Another final purification step includes high frequency resonant ultrasonic waves to separate cells instead of a physical mesh or membrane, which offers all the benefits of traditional devices but without their inherent problems and limitations. Technology based on acoustic resonance, is a non fouling / non clogging retention system offered by company Applikon from The Netherlands. A technique well within the reach of being disposable and hereby possible to introduce into the invented column.

### 5. Pumping capsules

The present invention comprises a capsule integrated reciprocating diaphragm pump based on single-use materials, single diaphragm pumping device, operating from absolute pressure to over atmospheric pressure, in its most simple form with electronically control by micro processor connected to various sensors and pneumatics operated valves, which are also present in the fluid streams. A preferred design of a pumping capsule is shown in figure 2.

Mostly the invented column set-up will require at least one capsule pump operating against either other pumps or flexible volume to compensate for pump pulses and volume change, such as:
1. One or more similar capsule pumps integrated in the column.
2. External bio container(s)
3. Internal storage volume, passive flexible element

In the present example the pumping capsule contains the electronics and pneumatics for pump and valve control. The capsules PCB may include provisions for internal data acquisition along the control power supply, which are all based on micro processors. The column capsules may integrate single-use sensors, wiring, and connectors in order to transmit data with the PCB. The column performance and integrity benefit from extended data acquisition capabilities from external mounted sensors of various specification and use.

The control unit capsule may further have provisions for control of stand-alone pumps, valves or other devices outside of the column or mounted on one or more of the flanges found on the capsule circumference. Such as valves for the supply of gases to the oxygen condition capsule for Oxygen concentration and pH control. Or such as the control of expandable tube valves in manifold capsules seen at figure 5. The pumping capsule or the manifold capsule may have provisions in order to remove entrapped gasses like air from the column start-up. The requirement of pressure relief valve is less of a problem as the fluid pressures are monitored on a real-time base at various location in the capsules, column.

Manifold capsules serve several purposes such as with active valves as seen in figure 5 comprising four identical valves of which two are in contact with the inner centre tube and the two other valves with the fluid ring tube around the centre. The expandable tube valves are inserted in the capsule body and integrated with flanges outside at the circumference. Valves shown here are pneumatic driven and needs to communicate with the pump & valve control capsule devices intended for such application.

The manifold capsule allows alternative drainage or feed, and access to fluids from the two centre fluid passages, which are not accessible by the pumping capsule.

### 6. Sensors incorporated in capsule

On-line and real-time measuring of O₂, pH, temp, CO₂, glucose, flow, pressures, cell counting sensors, etc. for monitoring and process control is mandatory for optimum production in the bioreactor. Any of the embodiments of the present invention may include passageways incorporated for correspondence with traditional electrochemical 12 mm diameter sensors probes for monitoring. Sensors as supplied from Hamilton, Mettler-Toledo, and others with Pg13.5 thread are used in the industry and inserted in ports via sealed connections.

Even though it is preferred to use single-use sensors and due to the fact that the industry shift to disposable bioreactors bags the need for disposable sensors also becomes important and a significant improvement. The above described bioreactor may be placed in a bag of flexible materials or in cassettes, capsules or the like from non flexible materials, which allow the bioreactor to be single-use and disposable. It is advantageous to avoid introduction of any foreign articles such as standard electrochemical 12 mm diameter sensors probes, transmitters after bioreactor sterilization. Such sensors will not accept the sterilization or they loose their calibration. Relevant real-time sensors are such as for those measurements of gases, dissolved gases, liquids, temperature, cell density, organics, pressures, etc.

The present invention benefits from disposable sensors which may be integrated into the bioreactor bag or capsule. Optical sensors have the advantages that they can be engineered to be non-invasive or introduced into the bioreactor before gamma sterilization occurs, do not have stringent grounding requirements, and can often provide enhanced performance over existing chemical or electrochemical methods. A variety of sensors principles are used; chemical-optical, optical, fluorescence, near infrared spectroscopic - NIR, Partial Least Squares - PLS, Principal Component Analysis - PCS, radio frequency impedance.

Compact single-use sensors are manufactured by the German company PreSens Precision Sensing GmbH being flat to less than 1-5 mm, less than 50 mm in diameter with wires or electrical connectors extending from the sensor. Or the sensor elements are attached to the inside of bioreactor corresponding through the translucent container material to a transmitter on the outside for non-invasive and non-destructive operation. An alternative product is manufactured by Finesse Corp from USA who offers a line of disposable optical sensors for detection of dissolved oxygen and pH.

### Operation mode

Opposite of the common standard batch fermentation the present invention by far takes advantages of continued fermentation.

In fed-batch mode, media and other nutrients are added at fixed intervals to the culture, and the product is only harvested at the end of the run. Batch fermentation reactor size vary from bench-top to 25 m³ tanks, operate for 7-90 days and collection of cells or harvest takes place only once per fermenter cycle. Foaming as to the mixer operation in traditional batch operation with a head space is a problem particularly if oxygen take-up is done through the fluid surface in the reactor. In general the cell density is low and CIP, "Cleaning In Place" after operation is highly costly and documentation heavy. This methodology is the most prevalent in the industry today; it is well understood and described, and produces higher yields than batch modes that do not involve feeds. Fed-batch systems are generally reliable; nutrients can be monitored and controlled, and media is used efficiently. As there is only one harvest, this leads to efficient process characterization, harvest of raw materials, and downstream processing. There are some potential disadvantages to the fed-batch method. Waste products such as lactate and NH4+ accumulate and the culture degenerates during the run. This can lead to the production of proteases and other degradation enzymes, which can act on the desired product. Also, key nutrients may become exhausted. Since the conditions within the culture may be constantly changing, cells go through several phases of growth, peaks, and troughs of production, and product may be degraded prior to harvest.

In perfusion mode, media and other nutrients are continuously exchanged and product is harvested throughout the culture period. Perfusion mode operation allow for continued fermentation and constant harvest. The bioreactor is operated in a configuration basically with one inlet and one outlet. The majority of the fluid flow volume is constantly passing the bioreactor capsule and re-circulated. A minor flow is continuously drained including the product and processed separately and outside of the bioreactor. A similar volume is added as fresh nutrient including oxygen addition. As no head space is required and the oxygenation is done outside the reactor foaming is not a problem. Thus, the process operates as a chemostat with cell retention, where the constant addition of fresh medium and the elimination of waste products provide the cells with the stable environment they require. This enables much higher cell densities to be achieved and, in turn, leads to higher productivity. Recent development results mentioned October 2007 in the magazine Genetic Engineering & Biotechnology News have shown that the ATF allows for relatively high cell densities. Cell numbers up to 7x10*⁷-x10*⁸ cells/mL with viability >90% are achieved on a regular basis with CHO cell lines, with perfusion flow limited to less than two volumes per day.

### Additional capsules

Prior text in the present description is concerned with specific capsule integrated devices. Other devices than those described may be integrated into the capsules for other specific purposes. Devices performing such as or including:
- Chromatography purification, affinity, ion exchange with membranes.
- Chromatography purification, ion exchange by packed beads. If capsules are filled with beads, gels, etc such capsules could also be of re-usable character and re-filled after use for circulation to the next production.
- Cross-flow, micro-filtration, ultra-filtration, membrane purification
- Bio catalysis reaction typically based on enzymes, to perform chemical transformations on organic compounds.
- Activated carbon based devices as to the high absorptive capacity suitable to treat fluid for removing colour, odour, etc.
- UVC irradiation, a short wave, germicidal wavelength that acts by causing dimerization and nicks in the DNA. The 254 nm wavelength is effective at inactivating viruses without damaging the proteins of interest.
- Ultrasonic wave separation support devices

In general all the invented capsules are pre-sterilized, of single-use character, which allow aseptic inter-connection in order to avoid cross contamination.

### Items of the present invention

### Detailed description to the figures

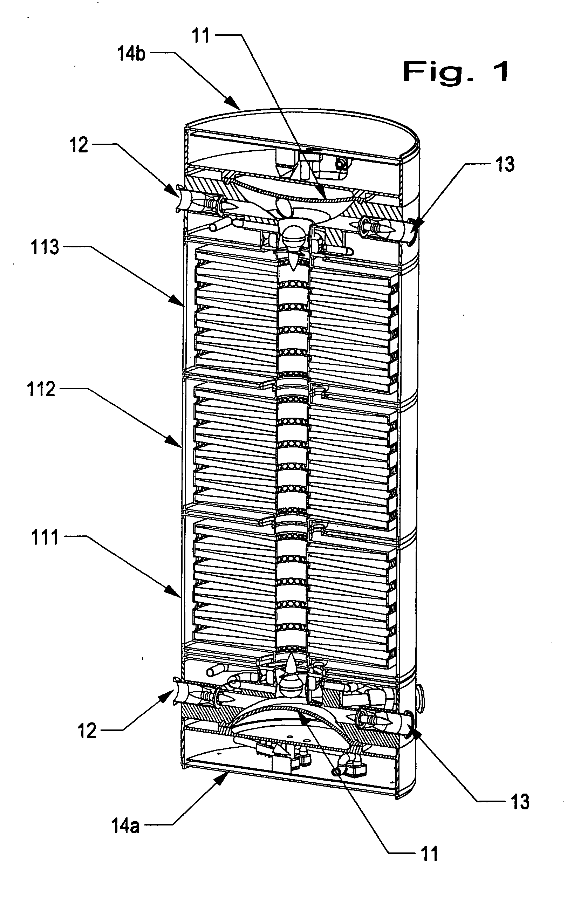

**Figure 1** illustrates the capsule assembling principle at its most simple configuration with non visible latches on the circumference frame for inter locking purposes. The configuration is shown in 3D perspective. Each capsule end-face is designed to exchange fluids with the neighbour capsule end-face. It is, however, also possible to exchange gases, electrical supply, data communication and sensor information via wires, hose integrated in the circumference wall. The capsules are of identical diameter (in all the illustration of Ø442 mm) for simple inter connection and preferably designed in several different heights named UH (one Unit Height in the illustration is 128 mm), such as:
- Bioreactor capsules (111, 112, 113) each being two UH
- Pumping capsules (14a, 14b) being one and a half UH

Each of the three bioreactor capsules (111, 112, 113) is identical and mounted independently of each other. As each capsule has a female and a male connector in the circumference an adaptor is needed between the bottom pumping capsule 14a and the bottom bioreactor capsule (111) to compensate for the fact that the bottom pump in capsule (14a) is turned opposite. Each of the two pumping capsules 14a, 14b have four identical valves (12a, 13a) and external ports (12, 13) (only half is visible) through the circumference and internal port to the diaphragm (11) fluid volume (11a). Further the pumping capsules (14a, 14b) house two integrated valves, which operate fully independently of each other in the capsule centre and connect the diaphragm volume to any other capsule fluid interface. All six valves in each pumping capsule (14a, 14b) operate fully independently of each other and are pneumatically controlled from the PCB below the diaphragm. More details are seen in figure 2.

No sensors are shown in Figure 1, but all sensors present are preferably of single-use capabilities. The illustrated set-up will work excellent in perfusion mode, when connected to an external supply of fresh media as well as to the purification devices. No external pumps are needed as the two integrated diaphragm pumps also handle the external circulation of fluids.

**Figure 2** illustrates a pumping capsule in 3D perspectives with the major four parts drawn apart from each other for better understanding. The same pumping capsule is also seen in Figure 1 and 9 in the present text.

Pump capsule primary disposable parts are:
1. rigid upper housing parts (20a) with four external media clamp connector flanges
2. rigid lower housing part (20b)
3. at least one flexible diaphragm (21)
4. four circumference tube sleeve valves of which (22a) and (23a) is visible
5. one set of end-face mounted sleeve rubber body valves
6. fluid control devices such as actuators for valves on PCB (60d)

Control capsule cover (20c) primary (not disposable) parts are:
1. micro processor and general electronics
2. sensors and their interface
3. pneumatic valves for control of six sleeve valves invisible under cover
4. proportional pneumatic valves (27c, 27c)
5. vacuum ejector pump (27)
6. connection for external supply of pressurized air, power supply and control interface

The pump capsule shell (20a) is a disposable housing parts manufactured from plastics. At least one circular end-face integrated dual element valve (29) with dimensions according to the purification capsules platform including both inlet and outlet. As to the advantage of fluid communication with purification and/or other capsules the axial dual valve elements (29a, 29b) opens or closes according to need for fluid access from the capsules connected to internal fluid volume (21 a). The upper housing part facilitate the central valve embodiment (29) and several valve embodiments (22a, 23a) pointing perpendicular to the central axis from said concave pumping chamber (21a) to the circumference of the upper housing part (20a) for external fluid communication. The valve bodies is elastic tube sleeves (23b), which inflated by pneumatic pressure added to outer side of the tube sleeve, force the tube to collapse around a central core part (23c) locking for any fluid flow through the valve. The lower housing part (20b) supports mechanically the pumping diaphragm (21) during maximum inflation with a concave upper housing part (20a) with the lower housing part (20b) for deflation further facilitating support for at least one displacement sensor (24), pressure sensor(s), and temperature sensor. Displacement sensor (24) based on laser principles able to measure the distance to and the position of the membrane (21) and absolute pressure sensors based on micro electronics, both with a flexible transparent window on the wall in order to separate the two different environments.

The control capsule (20c) contains on the circuit board (20d) the entire package of electronics, sensors and controls. Proportional valve(s) for diaphragm control, on/off valves for the tube sleeve valves, and micro processor including connections the driving gas inlet/outlet on the circumference.

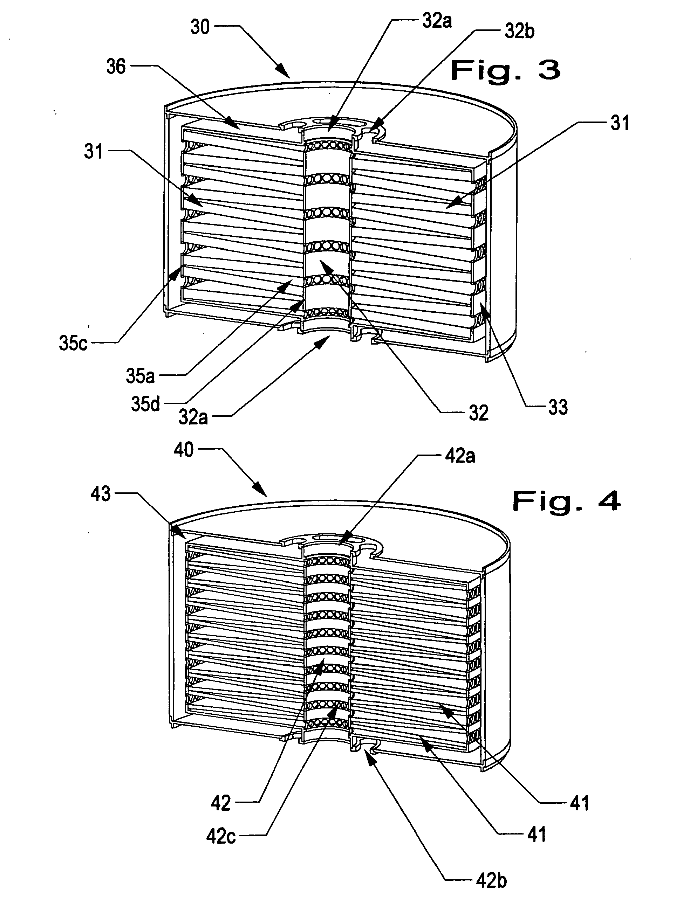

**Figure 3** illustrates a bioreactor capsule to be placed in a column. The bioreactor matrix disc capsules is situated inside a stackable capsule device (30) of rigid disposable materials with at least one fluid inlet (32a) and a series of symmetrically placed fluid outlets (32b) integrated axially within the design of capsules (30). Four radial oriented fluid outlet ports (32b) are connected to drainage collection volume (33) at the capsules periphery. The bioreactor capsule design comprises at least one set of matrix discs (31) and as here illustrated five sets of matrix discs (31). No flow spacers at the feed (32a) and drainage (35b) volumes between matrix discs (31) are shown for simplicity. Drainage spacers are connected to drainage collected volume (33) but not connected to the inlet volume (32) due to the presence of ring (35d). Feed spacer volume (35a) is not able to convey fluid to the circumference drainage collection volume (33) due to the presence of ring (35c). The fluid is forced to be transported from inlet volume (32) to discs (31) at its edge by ring (35d) and also restricted from exiting the discs (31) to the collection volume (33) by ring (35c). The forced fluid flow is introduced at one of two inlet (32a) filling up the inlet volume (32), and distributed to four full size and two half size feed spacer entrance (35a) radial filling the feed spacer volume by laminar flow at overpressure flowing perpendicular to the entire surface area of at least one matrix disc. The fluid then flows through the disc (31) to the drainage spacer volume (35b) and then further radial into the circumference collection volume (33), to be conveyed to end face collection volume (36) and outlet (32b).

More than one bioreactor capsule may be mounted on top of each other and work in parallel and be sealed by (30a) covering both inlet (32a) and exit (32b).

After the fluid has passed said bioreactor capsules the fluid may optionally pass further to at least one pumping capsule, a conditioning capsule, a separation capsule, etc. The illustrated capsules is 442 mm in diameter, one UH high = 256 mm high, but may take other dimensions, which may be required, and offer approx. 16 litre of matrix volume.

**Figure 4** illustrates a down stream processing capsule (40) comprising a number of traditional fibre based depth filter thin discs (41) oriented in sets angled to each other. In the present example eighteen identical discs (41) are positioned inside the capsule (40). On the circumference eight double step latches (not shown) are mounted and spread symmetrically around the capsule circumference for capsule assembling, which makes it easy to tightly keep the capsules together. Two dual fluid connections (42a, 42b) one at each end face are incorporated for connection to the neighbouring capsules. Fluid from the vertical inlet volume (42) guides flow through perforated tube (42c) to the inlet volume of the depth filter discs (41). Circumference collection volume for drainage fluids guides liquid to either of the end face flanges and through port (42b).

**Figure 5** illustrates a manifold capsule (50) having four identical expandable tube sleeve valves (56a, 56c), (56b and 56d are inside tubes in the capsule and hence not shown), which are operated by pressurized air supplied by a pump unit (not shown) via hoses (52h). On/off valves (not shown) either close or leave the valves open. Two external tri-clamp flanges connections (52a, 52b) are connected with the centre tube (55) and the other two flanges (52c, 52d) are connected with the fluid ring slot (54) around the centre tube (55). A rubber seal (57) insure leakage free connection to the interface similar neighbouring platform capsules.

**Figure 6** illustrates a conditioning capsule (60) with heating capabilities. The electrical heating elements are fully covered with approved materials and formed into four heating discs (61 a, 61 b, 61 c, 61 d) with fluid contact on each side, divided into two set (61a, 61b) and (61c, 61d) of discs in order to increase surface contact to the fluid that passes. Optionally the specific surface roughness or sets of ribs may be introduced in order to increase contact area and guide the fluid direction. Though in this version multiple circular slots (65) for fluid passage in the heating discs (61a, 61b, 61c, 61d) insures improved turbulence and contact between fluid and heating element discs. All heating discs (61a, 61b, 61c, 61d) are connected electrically to the plug (64) on the outside of the circumference of the capsule (60). Preferably at least one of the heating discs has a temperature sensor element integrated. Fluid inlet (61a) and fluid outlet (61b) may be interchanged, i.e. in some embodiments the fluid may enter the capsule through (61b) and leave the capsule through (61a). Two extra tri-clamps ports (63a, 63b) allow for various connections.

**Figure 7** illustrates a conditioning capsule (70) with symmetrically end face fluid connections and cooling capabilities. The stacked non porous wall tube heat exchanger coils (71) are located inside the capsule (70) with tube connection via two integrated tri-clamp flanges (74a, 74b) on the outside of the circumference of the capsule. The fluid flow path is labyrinth fluid flow created by three flat discs (75) between the coils. Two discs fixed to the centre inlet perforated tube, one disc fixed to the capsule circumference. Fluid inlet (71 a) is used on both sides to convey the fluid. Two extra tri-clamps (73a, 73b) allow for sensor connections. Either the tradition B.Braun type of Ø25 mm sensors or with a PG13.5 thread to tri-clamp adaptor also the smaller and more common 012 mm sensors become applicable. The capsule will also work as a sparging capsule (preferably combined with a bioreactor capsule with a need for oxygenation) with porous tubes coiled as shown and pressurized gases like air or oxygen inside the tube.

**Figure 8** shows a capsule concept, which by design is suitable for several purposes, such as for various surface filtration and separations or diffusion purposes. A series of parallel oriented semi permeable tubes or hollow fibres arranged tightly packed and at each end attached gas tight within, integrated within two (such as epoxy or the like) end covers leaving all the fibres open in each end. The complete unit fitted inside the capsule platform.

In the present illustration the capsule is an oxygenation capsule (80), which may be incorporated for the purpose of real-time adjustment of the oxygen content in the media as dissolved oxygen and/or as micro bubbles. The shown capsule integrates a capsule (81) for oxygen introduction, which is short and quite wide. The circular design is easy to integrate in the round capsule (80). Oxygen or air pressure is applied from the circumference and surrounds the multiple gas-permeable hoses/tubes/fibres (81a) between the epoxy end faces (81 b, 81c) and gas exchange, diffusion takes place through the fibre wall with the fluid in both directions. The media flows in axial direction with inlet from any of the end faces to the distribution chamber with access to the numerous numbers of gas-permeable tubes. The fluid are forced by pumping actions through the tubes to the opposite side and into collection chamber and further conveyed to the exit flange. Two connection flanges (83a, 83b) allow connection to valves (not shown) preferably controlled by the pumping capsules of either pneumatic or electric character. This feature makes it possible to adjust, in real-time, the supply of various gases and in particular oxygen, and the adjustment is controlled from within the column. The shown capsule offers a maximum of 5.7 m² surface area from 4,000 single hollow fibres tubes each being 05 mm and 90 mm long effective. The figure shows one single gas-permeable hoses/tube/fibre capsule, but in case of a relatively large diameter the capsule could be made up of a number of smaller units placed in parallel within the same capsule.

**Figure 9** shows the invented bio factory capsules stacked into a column (90) including the individual capsules as described above. The column comprises two bioreactor capsules (912, 913) similar to the capsule shown in figure 3, one down stream processing capsule (97) similar to the capsule shown in figure 4, three pumping capsules (94a, 94b, 94c) similar to the capsule shown in figure 2, two conditioning capsules (95, 96) similar to the capsules shown in figures 6, 7 and 8, respectively, and one manifold capsule (98) similar to the capsule shown in figure 5. The biofactory in figure 9 provides unlimited methods of use and includes specific connections in form of short hoses outside the stack between capsules, which facilitate the desired flow distribution along the column (90). An appropriate number of the capsules are equipped with external fluid connection flanges of tri-clamp design on the circumference. The illustrated set-up is appropriate for mammalian cell cultivation.

The column contains nine capsules and has a height of approx 1.7 meter. The single-use capability capsules are of identical diameter and platform concept in order to provide simple interconnection. The capsules are preferably designed in several different chassis heights in order to permit incremental capacity increase. Most of the capsules are able to perform bottom-up, to-down, bottom-in, and bottom-out. The easily interchangeable capsules in the bioreactor of figure 9 are:
1. Bioreactor capsules (911, 912)
2. Pumping capsules (94a, 94b, 94c)
3. Diffusion capsule for gas exchange and pH regulation (95)
4. Conditioning capsule (96) including three ports for additional sensors (96a)
5. Down stream processing capsule - in this example being one depth filtration capsule (97)
6. Manifold capsule (98)

The two bioreactor capsules (911, 912) are stacked on top of and next to the diffusion capsule (95) and together with the capsule below, (i.e. the temperature, fresh media and extra gas conditioning capsule 96), the passive production unit is provided. Below the temperature conditioning capsule 96 the pumping capsule 94a completes the active production unit, which is capable of operating independently of the down stream processing unit, which is made up of a down stream processing capsule (97) and a manifold capsule (98) and a pumping capsule (94c). On the circumference of the diffusion capsule (95) is mounted two flanges (95a, 95b) for access to gases such as CO2, air, and oxygen from an external supply to the inner core consisting of a hollow fibre capsule (95c). Fresh media in diluted or concentrated form may be introduced via one of several ports on either side of the conditioning capsules controlled by a valve. The two pumping capsules (94a, 94b) are able to service unlimited tasks of fluid communication requirements internal in the production unit as well as with external devices or any combinations thereof.

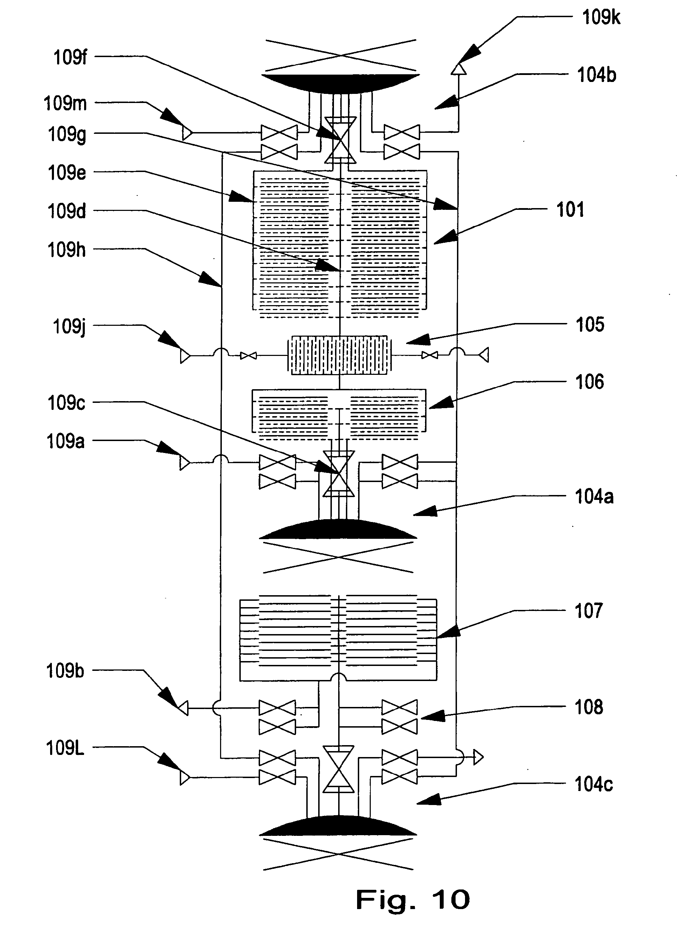

The down stream processing unit consist in the present illustration of one depth filtration capsule (97) on top of one manifold capsule (98) combined with one pumping capsule (94c). This independent down stream processing unit is operational on stand alone conditions also outside the figure 9 column. For simplicity only one down stream processing capsule (97) is shown. It is, however, possible to include several down stream processing capsules driven by the pump within the same column. Aseptic connection of two or more columns is done by sterile hoses externally via the generous use of tri-clamp flanges in order to perform the desired procedure of operation (not shown - see figure 10).

**Figure 10** shows a circuit diagram of the assembly in figure 9. Preferably all capsules integrate single-use sensors for monitoring at least oxygen temperature and pH values. The extra tri-clamp flanges on some capsules facilitate the use of any sensor.

The present figure illustrates the integration principle of the bioreactor capsule (101), which on the reactor top is connected to a in bottom-up position pumping capsule (104b) and on the reactor bottom side is connected to two conditioning capsules (105, 106), which are further connected to one pumping capsule 104a. On the rear side of each pumping capsules a pump control valve is located, which is shown as a wide black cross. Any of the interconnected pumping capsules (104a, 104b, 104c) is able to work simultaneously, in tandem, individually and without any restriction in timing and reasonable flow capability. The manifold capsule (108) is controlled by the pumping capsule (104c) control unit. Below the down stream processing capsule (107) a manifold capsule (108) connects the down stream processing capsule (107) with a port for drainage of product through valve (109b). The inlet to the down stream processing capsule (107) is positioned centrally at top of pumping capsule (104c) through the central of the manifold capsule (108). All fluid is fed through the pumping capsule (104a). Air drainage during start up is done through valve on pumping capsule (104b) via port (109k).

Fresh nutrient liquid is added through valve (109a) and sucked into the pump chamber of pumping capsule (104a), as the diaphragm is deflated, from an external volume corresponding to the product volume drained under pressure from pump (104c) through port (109b).

The dual in-line valve (109c) of the centre of pumping capsule (104a) conveys the main nutrient flow pressurized by inflating diaphragm in (104a) through the conditioning capsules (106) and (105) to the bioreactor capsule (101). Then the fluid passes the bioreactor (101) from inside volume (109d) to the circumference collection volume (109e) passing and through dual central valve (109f) located in pumping capsule (104b) while simultaneously the diaphragm in pumping capsule (104b) is deflated.

Inside the bioreactor (101) the flow is conveyed in a radial flow pattern from the central core inlet (1 09d), through the matrix discs for collection at the circumference volume (109e) and directed through the ring inlet valve part of dual central valve (109f) located in the pumping capsule (104b).

Nutrient liquid is re-circulated from pumping capsule (104b) through tube (109g) on the right side of bioreactor (101) to pumping capsule (104a) through an inlet valve, and the flow pattern as described above is repeated.

The down stream processing capsule (107) only collects a small amount of active micro organisms, because the majority of micro organisms are anchored in the bioreactor capsules (101). The down stream processing capsule (107) collects continuously dead cells and other particles as to the perfusion mode circulation and allows the purified product to pass.

A selected fraction of the retentate volume may be drained via drainage valve (109b) directly from down stream processing capsule (107), the retentate fluid volume being equivalent to the fresh nutrient volume being added at (109a). At desired sequences pumping capsule (104b) will direct fluid to pumping capsule (104c) through tube line (109h) and through central valve (109i), which forces the fluid to pass the down stream processing capsule (107) to drainage valve (109b) as product.

The conditioning capsule (105) receives pressurized sterile gases to be dissolved, and sparged into the fluid through valve (109j). The cross-flow conditioning capsule (106) contains temperature conditioning devices to insure correct bioreactor temperature.

Seeding with micro organism is done after the column is completely filled with media. The fluid is conditioned by re-circulation of the fluid for some time, which will insure collection of entrapped gas to be emptied via port (109k) by allowing pump (104a) or (104b) to suck the culture media into the pumping chamber and force the seeding fluid up through the conditioning capsule into the bioreactor for anchoring. Alternatively the seeding is performed through (109m) into pump (104b) and reversed directly down through centre of dual valve (109f) into the centre volume (109d) of bioreactor capsule (101).

The single-use, pre-sterilized, disposable capsule devices, which are assembled in column configuration, provide significant product expression improvements, very small foot-print, low-cost investment, elimination of CIP, SIP and offer easy of use. Further all capsules communicate with the common PLC for the whole stack with several close loop control algorithms performing the desired routines.

## Claims

1. A bioreactor factory device comprising a production unit for cultivating biological cells and for producing one or more chemical compounds, said production unit comprising at least three interconnected and stacked capsules, wherein said at least three capsules of the production unit is a bioreactor capsule, a first pumping capsule, which is a capsule integrated reciprocating diaphragm pump based on single-use materials and a second pumping capsule which is a capsule integrated reciprocating diaphragm pump based on single-use materials, wherein the first pumping capsule is positioned as the top capsule and the second pumping capsule is positioned as the bottom capsule in the stack of the production unit, said first pumping capsule and said second pumping capsule being in operable and liquid contact with each other and capable of regulating liquid to and from said bioreactor capsule.

2. The bioreactor factory according to claim 1, wherein the production unit further comprises at least one conditioning capsule located at any position between the first and the second pumping capsule.

3. The bioreactor factory according to claim 2, wherein at least one of the conditioning capsule(s) is a temperature regulating capsule.

4. The bioreactor factory according to claim 2, wherein at least one of the conditioning capsule(s) is a diffusion capsule.

5. The bioreactor factory according to any one of claims 1-4, wherein the bioreactor factory device also comprises a down stream processing unit, said down stream processing unit comprising at least one down stream processing capsule and at least one pumping capsule which is a capsule integrated reciprocating diaphragm pump based on single-use materials.

6. The bioreactor factory according to claim 5, wherein the production unit is positioned on top of the down stream processing unit and where a pumping capsule which is a capsule integrated reciprocating diaphragm pump based on single-use materials, is positioned as the bottom capsule of the down stream processing unit.

7. The bioreactor factory according to claim 5, wherein the down stream processing unit is positioned on top of the production unit and where a pumping capsule which is a capsule integrated reciprocating diaphragm pump based on single-use materials, is positioned as the top capsule of the down stream processing unit.

8. The bioreactor according to claim 5, wherein the down stream processing capsule is selected from the group consisting of a membrane filtration capsule, a separation capsule, an ion exchange capsule, a purification capsule, a chromatography capsule, an activated carbon capsule, and a packed bed capsule.

9. The bioreactor according to any one of claims 1-8, wherein the capsules are further provided with interlocking interface areas for exchange of fluid, electrical signals, electrical power, pneumatic signal exchange and/or vacuum signal exchange.

10. The bioreactor according to any one of claims 1-9, wherein the capsules are further provided with interlocking means, such as latches, for keeping together the capsules in the stack.

11. The bioreactor according to any one of claims 1-10, wherein the capsules in the stack are placed in the horizontal position next to one another or wherein the stack of capsules are placed in the upright position vertical to one another.

12. A down stream processing unit comprising at least two interconnected and stacked capsules, wherein said at least two capsules are one down stream processing capsule and one pumping capsule which is a capsule integrated reciprocating diaphragm pump based on single-use materials.

13. The down stream processing unit according to claim 12, wherein the down stream processing unit comprises at least a down stream processing capsule, a first pumping capsule which is a capsule integrated reciprocating diaphragm pump based on single-use materials and a second pumping capsule which is a capsule integrated reciprocating diaphragm pump based on single-use materials, wherein the first pumping capsule is positioned as the top capsule and the second pumping capsule is positioned as the bottom capsule in the stack of the down stream processing unit, said first pumping capsule and said second pumping capsule being in operable and liquid contact with each other and capable of regulating liquid to and from said down stream processing capsule.

14. The down stream processing unit according to any one of claims 12-13, wherein the capsules are selected from the group consisting of pumping capsules which is a capsule integrated reciprocating diaphragm pump based on single-use materials, separation capsules, filtration capsules, chromatography capsules, ion exchange capsules, storage capsules, conditioning capsules, sparging capsules, diffusion capsules, manifold capsules, valve capsules, column end capsules, and any combinations thereof, and wherein said capsules are capable of being stacked individually in any order and any number, with the proviso that the top capsule and the bottom capsule in the column must be pumping capsules which are capsules integrated reciprocating diaphragm pump based on single-use materials.

15. A method for cultivating microorganisms and producing a chemical compound by use of a biochemical reaction taken place in the bioreactor according to any one of claims 1-11.

16. The method for processing liquid from a bioreactor by use of a down stream processing unit according to any one of claims 12-14.

## Patentansprüche

1. Industrielle Bioreaktorvorrichtung, die eine Fertigungseinheit für das Züchten biologischer Zellen und für die Herstellung einer oder mehrerer chemischer Verbindungen umfasst, wobei die Fertigungseinheit mindestens drei miteinander verbundene und gestapelte Module umfasst, wobei die mindestens drei Module der Fertigungseinheit ein Bioreaktormodul, ein erstes Pumpmodul, das eine in das Modul integrierte Kolbenmembranpumpe auf der Basis von Einwegmaterialien ist, und ein zweites Pumpmodul sind, das eine in das Modul integrierte Kolbenmembranpumpe auf der Basis von Einwegmaterialien ist, wobei im Stapel der Fertigungseinheit das erste Pumpmodul als das oberste Modul angeordnet ist und das zweite Pumpmodul als das unterste Modul angeordnet ist, wobei das erste Pumpmodul und das zweite Pumpmodul funktionell und zum Flüssigkeitsaustausch miteinander in Kontakt stehen und in der Lage sind, die Zufuhr und den Abfluss von Flüssigkeiten zum und aus dem Bioreaktormodul zu regeln.

2. Bioreaktorvorrichtung nach Anspruch 1, wobei die Fertigungseinheit außerdem mindestens ein Aufbereitungsmodul an einer beliebigen Position zwischen dem ersten und dem zweiten Pumpmodul umfasst.

3. Bioreaktorvorrichtung nach Anspruch 2, wobei mindestens eines der Aufbereitungsmodule ein temperaturregulierendes Modul ist.

4. Bioreaktorvorrichtung nach Anspruch 2, wobei mindestens eines der Aufbereitungsmodule ein Diffusionsmodul ist.

5. Bioreaktorvorrichtung nach einem der Ansprüche 1 bis 4, wobei die industrielle Bioreaktorvorrichtung außerdem eine nachgeschaltete Verarbeitungseinheit umfasst, wobei die nachgeschaltete Verarbeitungseinheit mindestens ein nachgeschaltetes Verarbeitungsmodul und mindestens ein Pumpmodul umfasst, das eine in das Modul integrierte Kolbenmembranpumpe auf der Basis von Einwegmaterialien ist.

6. Bioreaktorvorrichtung nach Anspruch 5, wobei die Fertigungseinheit am oberen Ende der nachgeschalteten Verarbeitungseinheit angeordnet ist und wobei ein Pumpmodul, das eine in das Modul integrierte Kolbenmembranpumpe auf der Basis von Einwegmaterialien ist, als unterstes Modul der nachgeschalteten Verarbeitungseinheit angeordnet ist.

7. Bioreaktorvorrichtung nach Anspruch 5, wobei die nachgeschaltete Verarbeitungseinheit am oberen Ende der Fertigungseinheit angeordnet ist und wobei ein Pumpmodul, das eine in das Modul integrierte Kolbenmembranpumpe auf der Basis von Einwegmaterialien ist, als oberstes Modul der nachgeschalteten Verarbeitungseinheit angeordnet ist.

8. Bioreaktorvorrichtung nach Anspruch 5, wobei die nachgeschaltete Verarbeitungseinheit ausgewählt ist aus der Gruppe bestehend aus einem Membranfiltermodul, einem Trennmodul, einem Ionenaustauschmodul, einem Reinigungsmodul, einem Chromatographiemodul, einem Aktivkohlemodul und einem Festbettmodul.

9. Bioreaktorvorrichtung nach einem der Ansprüche 1 bis 8, wobei die Module außerdem mit verzahnten Schnittstellenbereichen für den Austausch von Flüssigkeiten und elektrischen Signalen, die Stromversorgung, den Austausch von pneumatischen Signalen bzw. den Austausch von Vakuumsignalen ausgestattet sind.

10. Bioreaktorvorrichtung nach einem der Ansprüche 1 bis 9, wobei die Module außerdem mit Verriegelungselementen ausgestattet sind, wie zum Beispiel mit einigen Riegeln, um die Module im Stapel zusammenzuhalten.

11. Bioreaktorvorrichtung nach einem der Ansprüche 1 bis 10, wobei die Module im Stapel in einer horizontalen Position nebeneinander platziert werden oder wobei die Module im Stapel in aufrechter Position vertikal aufeinander platziert werden.

12. Nachgeschaltete Verarbeitungseinheit, die mindestens zwei miteinander verbundene und gestapelte Module umfasst, wobei die mindestens zwei Module ein nachgeschaltetes Verarbeitungsmodul und ein Pumpmodul sind, das eine in das Modul integrierte Kolbenmembranpumpe auf der Basis von Einwegmaterialien ist.

13. Nachgeschaltete Verarbeitungseinheit nach Anspruch 12, wobei die nachgeschaltete Verarbeitungseinheit mindestens ein nachgeschaltetes Verarbeitungsmodul, ein erstes Pumpmodul, das eine in das Modul integrierte Kolbenmembranpumpe auf der Basis von Einwegmaterialien ist und ein zweites Pumpmodul umfasst, das eine in das Modul integrierte Kolbenmembranpumpe auf der Basis von Einwegmaterialien ist, wobei im Stapel der nachgeschalteten Verarbeitungseinheit das erste Pumpmodul als das oberste Modul angeordnet ist und das zweite Pumpmodul als das unterste Modul angeordnet ist, wobei das erste Pumpmodul und das zweite Pumpmodul funktionell und zum Flüssigkeitsaustausch miteinander in Kontakt stehen und in der Lage sind, die Zufuhr und den Abfluss von Flüssigkeiten zum und aus dem nachgeschalteten Verarbeitungsmodul zu regeln.

14. Nachgeschaltete Verarbeitungseinheit nach einem der Ansprüche 12 oder 13, wobei die Module ausgewählt sind aus der Gruppe bestehend aus Pumpmodulen, die jeweils eine in das Modul integrierte Kolbenmembranpumpe auf der Basis von Einwegmaterialien sind, Trennmodulen, Filtermodulen, Chromatographiemodulen, Ionenaustauschmodulen, Speichermodulen, Aufbereitungsmodulen, Gasverteilermodulen, Diffusionsmodulen, Sammelrohrmodulen, Ventilmodulen, Säulenabschlussmodulen und beliebigen Kombinationen davon, und wobei die Module in der Lage sind, einzeln in einer beliebigen Reihenfolge und einer beliebigen Anzahl gestapelt zu werden, unter der Bedingung, dass das oberste Modul und das unterste Modul in der Säule Pumpmodule sein müssen, die jeweils eine in das Modul integrierte Kolbenmembranpumpe auf der Basis von Einwegmaterialien sind.

15. Verfahren zum Züchten von Mikroorganismen und zur Herstellung einer chemischen Verbindung unter Verwendung einer biochemischen Reaktion, die in einem Bioreaktor nach einem der Ansprüche 1 bis 11 stattfindet.

16. Verfahren zum Verarbeiten einer Flüssigkeit aus einem Bioreaktor unter Verwendung einer nachgeschalteten Verarbeitungseinheit nach einem der Ansprüche 12 bis 14.

## Revendications

1. Dispositif pour usine avec bioréacteur comprenant une unité de production pour la culture de cellules biologiques et pour la production d'un ou de plusieurs composés chimiques, comprenant ladite unité de production au moins trois capsules verrouillées et empilées, dans lequel lesdites au moins trois capsules de l'unité de production sont une capsule de bioréacteur, une première capsule de pompage, qui est une pompe de membrane oscillante intégrée dans la capsule et basée sur des matériaux d'usage unique et une deuxième capsule de pompage qui est une pompe de membrane oscillante intégrée dans la capsule et basée sur des matériaux d'usage unique, dans lequel la première capsule de pompage est placée en tant que la capsule supérieure et la deuxième capsule de pompage est placée en tant que la capsule inférieure dans la pile de l'unité de production, étant ladite première capsule de pompage et ladite deuxième capsule de pompage en contact opérationnel et liquide l'une avec l'autre et étant chacune capables de réguler l'écoulement de liquide vers et provenant de ladite capsule de bioréacteur.

2. Usine avec bioréacteur selon la revendication 1, dans laquelle l'unité de production comprend en outre au moins une capsule de conditionnement placée dans une position quelconque entre la première et la deuxième capsule de pompage.

3. Usine avec bioréacteur selon la revendication 2, dans laquelle au moins une des capsules de conditionnement est une capsule de régulation de la température.

4. Usine avec bioréacteur selon la revendication 2, dans laquelle au moins une des capsules de conditionnement est une capsule de diffusion.

5. Usine avec bioréacteur selon une quelconque des revendications 1 à 4, dans laquelle le dispositif pour usine avec bioréacteur comprend en outre une unité de traitement placée en aval, comprenant ladite unité de traitement placée en aval au moins une capsule de traitement placée en aval et au moins une capsule de pompage qui est une pompe de membrane oscillante intégrée dans la capsule et basée sur des matériaux d'usage unique.

6. Usine avec bioréacteur selon la revendication 5, dans laquelle l'unité de production est placée au sommet de l'unité de traitement placée en aval et dans laquelle une capsule de pompage qui est une pompe de membrane oscillante intégrée dans la capsule et basée sur des matériaux d'usage unique est placée en tant que la capsule inférieure de l'unité de traitement placée en aval.

7. Usine avec bioréacteur selon la revendication 5, dans laquelle l'unité de traitement placée en aval est placée au sommet de l'unité de production et dans laquelle une capsule de pompage qui est une pompe de membrane oscillante intégrée dans la capsule et basée sur des matériaux d'usage unique, est placée en tant que la capsule supérieure de l'unité de traitement placée en aval.

8. Usine avec bioréacteur selon la revendication 5, dans laquelle l'unité de traitement placée en aval est choisie dans le groupe constitué d'une capsule de filtration à membrane, une capsule de séparation, une capsule d'échange d'ions, une capsule de purification, une capsule chromatographique, une capsule de charbon activé, et une capsule de lit cassé.

9. Bioréacteur selon l'une quelconque des revendications 1 à 8, dans lequel les capsules sont dotées en outre avec des régions d'interface de verrouillage pour l'échange de fluide, l'échange de signaux électriques, l'alimentation en courant électrique, l'échange de signaux pneumatiques et/ou l'échange de signaux de vide.

10. Bioréacteur selon l'une quelconque des revendications 1 à 9, dans lequel les capsules sont dotées en outre de moyens de verrouillage, tels que des loquets, de façon à tenir ensemble les capsules dans la pile.

11. Bioréacteur selon l'une quelconque des revendications 1 à 10, dans lequel les capsules empilées sont placées dans sa position horizontale côte à côte ou dans lequel les capsules empilées sont placées dans sa position verticale l'une sur l'autre.

12. Unité de traitement placée en aval comprenant au moins deux capsules interconnectées et empilées, dans laquelle lesdites au moins deux capsules sont une capsule de traitement placée en aval et une capsule de pompage qui est une pompe de membrane oscillante intégrée dans la capsule et basée sur des matériaux d'usage unique.

13. Unité de traitement placée en aval selon la revendication 12, dans laquelle l'unité de traitement placée en aval comprend au moins une capsule de traitement placée en aval, une première capsule de pompage qui est une pompe de membrane oscillante intégrée dans la capsule et basée sur des matériaux d'usage unique et une deuxième capsule de pompage qui est une pompe de membrane oscillante intégrée dans la capsule et basée sur des matériaux d'usage unique, dans laquelle la première capsule de pompage est placée en tant que capsule supérieure et la deuxième capsule de pompage est placée en tant que capsule inférieure dans la pile de l'unité de traitement placée en aval, étant ladite première capsule de pompage et ladite deuxième capsule de pompage en contact opérationnel et liquide l'une avec l'autre et étant capables de réguler le flux de liquide vers et provenant de la capsule de traitement placée en aval.

14. Unité de traitement placée en aval selon l'une quelconque des revendications 12 ou 13, dans laquelle les capsules sont choisies dans le groupe constitué des capsules de pompage étant chacune une pompe de membrane oscillante intégrée dans la capsule et basée sur des matériaux d'usage unique, des capsules de séparation, des capsules de filtration, des capsules chromatographiques, des capsules d'échange d'ions, des capsules de stockage, des capsules de conditionnement, des capsules d'aspersion, des capsules de diffusion, des capsules d'admission, des capsules de soupape, des capsules d'extrémité de la colonne, et des combinaisons quelconques de celles-ci, et dans laquelle lesdites capsules sont capables d'être empilées individuellement dans un ordre et nombre quelconque, à condition que la capsule supérieure et la capsule inférieure dans la colonne soient des capsules de pompage étant chacune une pompe de membrane oscillante intégrée dans la capsule et basée sur des matériaux d'usage unique.

15. Méthode de culture de microorganismes et de production d'un composé chimique en utilisant une réaction biochimique ayant lieu dans le bioréacteur selon l'une quelconque des revendications 1 à 11.

16. Méthode pour la traitement de liquide provenant d'une bioréacteur en utilisant une unité de traitement placée en aval selon l'une quelconque des revendications 12 à 14.
